# EUROPEAN PATENT SPECIFICATION

(11) **EP 1 614 426 B1**
(45) Date of publication and mention of the grant of the patent: **17.12.2008**
(21) Application number: 04712469.8
(22) Date of filing: 19.02.2004
(51) Int. Cl.: A61K 39/106, C12N 7/00, C07K 14/28, A61P 37/04

(54) **ATTENUATED STRAINS OF VIBRIO CHOLERAE AND LYOPHILISED VACCINES CONTAINING SAME**
ABGESCHWÄCHTE STÄMME VON VIBRIO CHOLERAE UND DIESE ENTHALTENDE LYOPHILISIERTE VAKZINE
SOUCHES ATTENUEES DE VIBRIO CHOLERAE ET VACCINS LYOPHILISES CONTENANT CES SOUCHES ATTENUEES

(30) Priority: 20.02.2003 CU 3003903; 17.04.2003 CU 3008403
(43) Date of publication of application: 11.01.2006
(73) Proprietor: Centro Nacional De Investigaciones Cientificas (CNIC), Ciudad de la Habana 12100 (CU)
(72) Inventor: CAMPOS GOMEZ, Javier, Villa Clara, Santa Clara 50 100 (CU); MOREIRA HERNANDEZ, Tomás Marcelino, La Lisa, Habana 11 500 (CU); RODRIGUEZ GONZALEZ, Boris Luis, San Agustin, La Lisa, Habana 13 600 (CU); MARRERO DOMINGUEZ, Karen, Fontanar, Boyeros, Habana 19 250 (CU); MARTINEZ GUTIERREZ, Eriel, Playa, Habana 12 100 (CU); LEDON PEREZ, Talena Yamilé, Centro Habana, Habana 12 400 (CU); SILVA LARRANAGA, Yussuan, Plaza De La Revolucion, Habana 12 300 (CU); SUZARTE PORTAL, Edith, Mantilla, Arroyo Naranjo, Habana 13 800 (CU); DELGADO RODRIGUEZ, Herminia de la Caridad, Plaza De La Revolucion, Habana 10 400 (CU); URRA VILLAVICENCIO, Caridad, Marianao, Habana 11 500 (CU); FANDO CALZADA, Rafael Alfredo, Reparto Flores, Playa, Habana 12 100 (CU)
(74) Representative: Carpintero Lopez, Francisco
(86) International application number: PCT/CU2004/000002
(87) International publication number: WO 2004/073736

(56) References cited:
- WO-A2-99/55348
- JOURAVLEVA E.A. ET AL.: 'The Vibrio cholerae mannose-sensitive hemagglutinin is the receptor for filamentous bacteriophage from V. cholerae O139' INFECTION AND IMMUNITY vol. 66, no. 6, June 1998, pages 2535 - 2539, XP002992814
- EHARA M. ET AL.: 'Characterization of filamentous phages of Vibrio cholerae O139 and O1' FEMS MICROBIOLOGY LETTERS vol. 154, no. 2, 15 September 1997, pages 293 - 301, XP002992815
- VALLE E. ET AL.: 'Construction and characterization of a nonproliferative E1 Tor cholera vaccine candidate derived from strain 638' INFECTION AND IMMUNITY vol. 68, no. 11, November 2000, pages 6411 - 6418, XP002992816
- PAVELKA M.S. JR & JACOBS W.R. JR: 'Comparison of the construction of unmarked deletion mutations in Mycobacterium smegmatis, Mycobacterium bovis Bacillus Calmette-Guerin, and Mycobacterium tuberculosis H37Rv by allelic exchange' JOURNAL OF BACTERIOLOGY vol. 181, no. 16, August 1999, pages 4780 - 4789, XP002992817

### SEQUENCE LISTING

<110> Centro Nacional de Investigaciones Cientificas
<120> Attenuated strains of *Vibrio cholerae* with improved biological safety features in freeze dried form for oral vaccination
<130> VGJphi
<141> 2004-02-20
<150> CU 2003-0039
   <151> 2003-02-20
<150> CU 2003-0084
   <151> 2003-04-17
<160> 1
<170> Patent version 3.1
<210> 1
   <211> 7542
   <212> DNA
   <213> Filamentous phage of *Vibrio cholerae*
<220>
   <221> ORF359 CDS
   <222> (1)..(1077)
   <223> Similar to the replication protein, pII, from other filamentous phages
<220>
   <221> ORF 112 CDS
   <222> (1085)..(1420)
   <223> Hypothetic Protein
<220>
   <221> ORF81 CDS
   <222> (1433)..(1675)
   <223> Hypothetic Protein
<220>
   <221> ORF44 CDS
   <222> (1690)..(1821)
   <223> Similar to the major protein from the Capsid, pVIII, from other filamentous phages
<220>
   <221> ORF29 CDS
   <222> (1839)..(1925)
   <223> Hypothetic Protein
<220>
   <221> ORF493 CDS
   <222> (1954)..(3432)
   <223> Similar to a minor protein from the Capsid, pIII, from other filamentous phages
<220>
   <221> ORF80 CDS
   <222> (3510)..(3749)
   <223> Hypothetic Protein
<220>
   <221> ORF384 CDS
   <222> (3755)..(4906)
   <223> Similar to a protein of the assembly, pI, from other filamentous phages
   <220>
   <221> ORF104 CDS
   <222> (5232)..(5543)
   <223> Similar to a protein of the assembly, pI, from other filamentous phages
   <220>
   <221> ORF67 CDS
   <222> (7328)..(7528)
   <223> Hypothetic protein
<220>
   <221> ORF 136 CDS
   <222> (6105)..(6112)
   <223> Complementary chain
   Putative transcriptional regulator I
<220>
   <221> ORF154 CDS
   <222> (6439)..(6900)
   <223> Complementary chain
   Putative transcriptional regulator I
<220>
   <221> ORF58 CDS
   <222> (6439)..(6900)
   <223> Complementary chain Hypothetic protein
<400> 1

### SEQUENCE LISTING

<110> Centro Nacional de Investigaciones Cientificas
<120> Attenuated strains of *Vibrio cholerae* with improved biological safety features in freeze dried form for oral vaccination
<130> VGJphi
<141> 2004-02-20
<150> CU 2003-0039
   <151> 2003-02-20
<150> CU 2003-0084
   <151> 2003-04-17
<160> 1
<170> Patent version 3.1
<210> 1
   <211> 7542
   <212> DNA
   <213> Filamentous phage of *Vibrio cholerae*
<220>
   <221> ORF359 CDS
   <222> (1)..(1077)
   <223> Similar to the replication protein, pII, from other filamentous phages
<220>
   <221> ORF 112 CDS
   <222> (1085)..(1420)
   <223> Hypothetic Protein
<220>
   <221> ORF81 CDS
   <222> (1433)..(1675)
   <223> Hypothetic Protein
<220>
   <221> ORF44 CDS
   <222> (1690)..(1821)
   <223> Similar to the major protein from the Capsid, pVIII, from other filamentous phages
<220>
   <221> ORF29 CDS
   <222> (1839)..(1925)
   <223> Hypothetic Protein
<220>
   <221> ORF493 CDS
   <222> (1954)..(3432)
   <223> Similar to a minor protein from the Capsid, pIII, from other filamentous phages
<220>
   <221> ORF80 CDS
   <222> (3510)..(3749)
   <223> Hypothetic Protein
<220>
   <221> ORF384 CDS
   <222> (3755)..(4906)
   <223> Similar to a protein of the assembly, pI, from other filamentous phages
   <220>
   <221> ORF104 CDS
   <222> (5232)..(5543)
   <223> Similar to a protein of the assembly, pI, from other filamentous phages
   <220>
   <221> ORF67 CDS
   <222> (7328)..(7528)
   <223> Hypothetic protein
<220>
   <221> ORF136 CDS
   <222> (6105)..(6112)
   <223> Complementary chain
   Putative transcriptional regulator I
<220>
   <221> ORF154 CDS
   <222> (6439)..(6900)
   <223> Complementary chain
   Putative transcriptional regulator I
<220>
   <221> ORF58 CDS
   <222> (6439)..(6900)
   <223> Complementary chain Hypothetic protein
<400> 1
   atgatacggt tcgccctgtc aaagttgacc acttggcttt tactttcgcc tatgcggact 60
   tgcgccactt ggacaaaagc aacgaccaag actttatcaa tctacagatg cccgtttatc 120
   acgagccaaa gacccgaacc aaggaacaag gcgcggtgtg ctctaccttg gaacaaatcg 180
   agcatcatat ggaagcgcac cgaaacaaag tgtccaagat gctctttcat cgctttgatt 240
   tgttcatgtc caaaatcatg gctttcgtta tcgcctatgc gtggtcgtgg ccttcatggt 300
   tacaacgatt ctatggtcat tctcgatatg accggacaag ttgagtgtgg ccttgtcgga 360
   attggcggaa acaacgatac cgtttttgtc caaatcaacg gcacggggtg caccaaactt 420
   ttcgaccgta tcgactctaa gaagcttcat tggtggctcg ctcaggttct tggcattact 480
   cgcttagttc gtctcgactt ggccgtggac gattacaccg gaaacttcga cgccaagtat 540
   gcagagaaat gtttttatga gggagcattt cgcactgctc cacgggggca aggtccctca 600
   atggttcctc ataaacgcat tacagaaaac ggcgctttga tggaagaagc aacgattgtc 660
   ggctctcgtt cctcggcgat ttactggcgt atctacaaca aaaagcttga gcaaaaaatt 720
   actgaccctg acctgatttg gtatcgaaac gaggttgagc tgaaaaagtg cgacatcgag 780
   cttttagcca atcctgccgc ctcttttgcg ggtatctgcc ctttcgcggc ctctatcgag 840
   tgtacgcctc cggttaagtt ctctcgcaac aaaaaggctc aaggtcttga atttatggct 900
   cgcatcgcat gggttcgccg tcaatgtggc gtggcgttag cggaagttat cgccatgacg 960
   caaggcgatt taggcgaagc attcgggatg cttatccctc acaaacatag acgccctgac 1020
   tttgaattgc tcggcgttcc tgattcatac acacaactga aaaacacact atggagttaa 1080
   ggtaatggct aacatcactg gcatcgtcat caaaacattt cctaaatcgg gtaccacgat 1140
   tgcagagctg aacgttcttc gccctgttga aaccgtcaac gttgagaagt ttgctcaata 1200
   cggtttgggg ctaaacacgg atattccttt caataagcag ccgctgcgta tcgaacctac 1260
   ttacgccaag cgtttgattg aaacacgcgc ttttgttcct aaccgtgaat atgacattcg 1320
   ctttggtagt aaccctgacg acccattgga agtcgttgct gttgagctca tccccaagga 1380
   tgacgacctt aaaaaatact ttactgaaac acttaaaaag taaggaagtt ttatgcctgt 1440
   gtgcgctctc ccaaatagtc agggattcct agcggttact gacaagccac tgaatgaatg 1500
   tgacggcggt tatgttgctt tcactattca ggattacgac tacttgatga gctacacacg 1560
   cataacccca accgatgccg gaacggcctt tagcttcggt tttatggctg tattcgctct 1620
   cggttattta tatacctatg ccgtttatat cggtaaaaaa ctcatcaacc tcttataagg 1680
   agatacatca tggctgatat ctttggcgca attgattttg caggtgtcgc tgctcttgtt 1740
   actgctgctg gtgttgccat cattggcatt actatggctt tcaaaggcat ctcacttggc 1800
   aaacgcgctg ttaacaaagc ctaatcggta actgaactat gcttattgcc ctgcatgata 1860
   ttcagttaat tattttcgcg ttgctgggtg gcatgtcggg ttttatagct gctctcaact 1920
   tccgataaca aaggggctaa cgcccctttt tttatggtta tgtttatgcg caaatttttt 1980
   attatctctc ttgtcattag cttatatttc accgttcttc ctgcttttgc tgcttatcag 2040
   ttaccttttg accaaacaaa gactttctca actgttcagg ccgccgctga gtattatgtc 2100
   aatttacttg gtggtacttc atgcaaggct gacggtaaca ggtttaaaat ttacagagtt 2160
   aaatctattg gcgacccctc ttttgttatt caacaagaaa cctatttaga caacaaatgt 2220
   caggtctttt catcaaaggg tgatatcagc gttactctta ttgttgttga cgaccctacc 2280
   acctgcgagg cttcaaaagg tcaaacagga aaagtcggtt ggaattctta cttctggggc 2340
   tcggcaactc catcccgtta tatctgctct tctgcttatg gtggttgtgt tgccctcact 2400
   ggtgaccatt tatgtattaa tattgaccct gaagcattga aaaatgaccc gtctctttat     2460
   gattgcgatg ctctctatat cgttcaggat actccttgtt ccccttctgg tgattatcca    2520
   ttttgtaccg atgagaattg ctcttctttt cttcctgagc cgaatcctaa ccctaaccct 2580
   gaaccagagc cggagccaga accccaacca gacccagaac acaatccctc tgacccgaca 2640
   gcgccactcc ctggctctgg cggtgaagtc attaacccta ctgttcctcc taaaccaccg 2700
   accgaggaaa cgcctaagcc tgacgtagaa acaccagacc ctacacctga ttcaaattct 2760
   gacgttgttc aatctgttac cggaatgaat gaggatatga acgagttatt aactcgactc 2820
   aattctgaca acaacaagca gcttgatgat gttaacaatc agctcttgca gctcaataca 2880
   caatcacaac gtatcgttgc tcagattgcc aaacaggaaa aacaagatgc tgccatctac 2940
   gaaaatacaa aggcacttat ccagaacctt aacaaagacg tgaccacggc cgttaacaaa 3000
   accaccaatg ccgttaatgc tttgggctct aaagttgatg gcttatccga tgccgtcgat 3060
   ggtcttggtg aagatgtatc agcaataaaa gatgtaatta ctaatgttga tacttctggg 3120
   gctggtattt ctggcacttg tatagagtct gacacttgta cggggtttta tgaatctggt 3180
   tatcctgatg gtatttcagg catattttca cagcattttg aaatcgtctc tgaaagtgtt 3240
   actgataccg tcaaagactt tatgaaaatt gatttatctc acgctcaaag gccatcattt 3300
   tctattccag ttctccactt tggaaatttc agctttgacg attatataaa ccttgattgg 3360
   atatttggct ttgttcgtgt ttgcatgatg gtttcaactg ctttcctatg tcgtaaaata 3420
   atattcggag gttaatatgg attgggtaat tgatttattt aaccaagctt atcgagtttc 3480
   tttaccgctt attaatcacg cttattgata tgctaaagga tgttttcttg tggcttattg 3540
   atggcgttct ttctgccgta aaccttttat tagagaaagc attatcactc atagaaccaa 3600
   tggatgtttc ctcttacttg actggaatcc cttccggtgc tgcttgggtt attagcgcca 3660
   ttggtatccc tcagtgtctt ggtatgatta tgtcagcaat cattgttcgt atcttattgc 3720
   aactcgttcc attcactcgt ttaggttctt aattatgatt tatgcaattg ttggccgtcc 3780
   tcgctctggt aagtcatacg agtctgttgt ttatcatatt attcccgccg ttaaatctgg 3840
   ccgtaaagtc attacaaata ttcctttaaa tatggatatg tttgtaaagg ttttcggtga 3900
   ttcggttaga gatttaatcc gcatcgtcga tgctaaattt aatgaatacg gttcaatgaa 3960
   tagaccattt tcaaaggttg atgattatct tgatgactgg cgagatgata aaaaccgcgc 4020
   tcctctttat gttattgatg aggctcatat ggttattcca actcgcctcg gtgatcaaag 4080
   atacttgagt tctattcaat gcacggttca ttacggcatc gatattatta ttctcactca 4140
   aaatttaaga aagattcatg ctgatattag agcaatgatt gagatgactt attactgtgc 4200
   taaaaatacc gcattcggca gtaaaaagac ttatacaaaa aaggttcgca tcggtgatac 4260
   cagagaagac ttacacatag agcaacgcac ttacaaagaa cactatttcg gtttttatca 4320
   atctcatact caaagcgcag gctctgtagt cgaagctcag gcattcgaca ttactcccat 4380
   ttggaagcgc tggcctttct ggggctctct tgtctgcttc attatcgtta ttcttatact 4440
   tgcttattat tttcagtcac gtaagtcaaa acacgctgaa gatgttcccc cagttcctga 4500
   gcacagccaa cagactcctt catctgactc atccatttct caccctccta aacctcttca 4560
   aaccataaag gctgctcctc tctctgagcc gctaagggat tttcagcttt acgtatctgg 4620
   ccacgctaag cagatagcct ataaaaagat gtcattttct cgagagattg ataccaggct 4680
   aaccttctat cacgtttaca ttagcgctta tcaggatgac aaattctctt tctctctcaa 4740
   taacatagac ttagaaaaga tgggttatca atttgaggct ttaacggagt gcgtttatcg 4800
   tattacttgg ggctctaatt ctcgtgtcat tacctgtatt gatgaaagcc gtttcaatca 4860
   gcaaaaggcc gaaactgtat tcgaccacgt tcctaaactt gatatttgaa aagcgtttca 4920
   gacttcaaac atcttgatta ttgaaacact tttcaaggaa tttgatttgt gtaacagatt 4980
   tcatgacctt gattgctgta acatatatca agttacttga aatctgttac aggaatcaat 5040
   tatgtcttat tgcaaatctt ctttcccaga ggctgatttt gctgtttatc aggagttcca 5100
   aagaaagctt ggcccctaaa tataaatcta gaccagaggc cccctattag ccttgctcgc 5160
   caatatatgg agaaataccc tgagtttcag cacattggcg gcattggtac acctctcgat 5220
   gttattgacg aatgcttaga gcagattgct gcccttcaaa agcccaaaca cggaggaaac 5280
   gcaagggcgc tgcccgtaag cctgcaccaa gtcaacttaa gcgcgttcct gttcctctca 5340
   tcccaatcat tgaccgtatc atctcccaat ataaatcttg tgacttaaca tctttttcgc 5400
   ttcttgcctt atatgattat ctagttactc aaagtttcag cctgtgcgag ggttctgttt 5460
   ttaaaggtct ttttcttgat gatgttactt ttgaaccgtt agatgaccct gaacataatc 5520
   ccccttcgat acaaaaaggt ttctgactac tttcttcaag ctggcctact tgatgacttt 5580
   tacaattggg ctatctccaa cggccgcctt gctctttagc tttttaattt tctttattaa 5640
   ctatccgcgc cttagtgcgc gacttttgag cttagctcga tgcccgcagg gactaggcca 5700
   taacgttagc ctcaacacac tttgaacgta tgctactgca tatccgaaac actctaaggt 5760
   tcgcggcggt tcgcttgatt ctaaagcgcg ccagtcagtc aagtgatcat caatactctt 5820
   gcgccagaaa aaacaccctt cgccctgcca agccataaaa gatgtttcag caagcgcagt 5880
   aggtagcagc attttctcgc cgtactatca acaacagggc gcggcgagtg tcgagcaagc 5940
   catatttcat tcgttaagcg ttgcgctctg gaggggccca ctgggaggac acgggaggaa 6000
   gcgccaaccc ccgagctgta tcacgggggt agattccacc atactccttg gtctcaccgt 6060
   ataaatccct aaaaaaaatt agggctactg cctccctgct tttacttaat ccctttgatt 6120
   ctagccatag ctctagcgta cttgagaagt ttagaacgtg tcattgcatc gtctggtgct 6180
   tgtatttgca agatagcgat agcggctaat aactgctgtg gtgctaccct gtcacctgtt 6240
   gggaatatca gtctcccccc ttccatccta aatccccacc actcatcacc gtaatacagc 6300
   tcttttctgc tgtgccatct catcagcctc ttgcaaatcg gtggtatctt ctcccctgcg 6360
   tcccattgct tgacctcgct cacagtttta aaacaaagtt tcgccgcttc ttcaacgctt 6420
   aacccgcatt caaattcacg aaaaacaaaa ttctttgtca tttcgcttcg attcattgat 6480
   aaaactccca aaagcggaag ttttataagc atttgaattt atagcaactt ttaccataag 6540
   ccgacataat gcgaagtaag aaacagcctc gttaaactgg ctgattgaca ccgctagacc 6600
   accgcaaagc attgatattg ttcttaatcc tagcccgttt tgcttttttg ctatgctttc 6660
   ccaaatcgct ttgatgcgtg ggttttcgtt gcggtctgcg tgacatccta acagtgcgat 6720
   ttcagggtca attcctgctg attcagctaa aaaaattgct tcttcatcag atatatacct 6780
   aactccagtt ctcattttgc ttattttctg tggcgacaag ttcaagtcgt gtgcaatttg 6840
   cttgtcttgt acgtagtttt ttgccttttt gtaggcgtct aacagttcat ttgcatacat 6900
   atttatccct ccttttcttt tctatcatag cttgccaatc cccaatttgc gctatttaca 6960
   atccccaaaa atgggactag aatcctcata aatcggaatt tgaccacctt gggcgctaga 7020
   ccttaactct tccccttggt ggtctcccca accagttaag gcggttatca tggcaaattc 7080
   agcaaagaaa caaaccctat cacaatctgt taacccattt gtgaccatcc agttaacttc 7140
   tggcgctctt cctcgctttc ttgcttacgg cggctttaat tcggatggtt ctcagcgctc 7200
   taagtaactt ctgtttctac tgatgcccca caaaatgccg cttcctactg caaacgcctt 7260
   ttacccaagg ataagcgtaa ttggcctcta gctcagattt cctatcaggt taaataaggt 7320
   cgctatcatg ggtgatttca tctactacga caacgaaccc aacatcggga tcaacgtgta 7380
   tttcgtttgg gggcatcgtt tctttaaaaa ctggcctgag ttagagcaat accttgccat 7440
   tcactatggc gctgacccat atcaactggt tgaaatcact aacgaaaact acaacgaatt 7500
   gcttttaaag ggggtctttc atgccatgta agcaccctca cc 7542

## Description

### Technical sector.

The field of invention is that of biotechnology, in particular, the obtainment of *Vibrio cholerae* live attenuated vaccine strains, more specifically, the introduction of defined mutations to prevent or limit the possibility of reacquisition and (or) the later dissemination of CTXΦ phage encoded genes by those live vaccine strains and a method to preserve them to be used as vaccines.

### Previous art

### First, definitions:

During the description of the invention will be used a terminology whose meaning is listed bellow.

By CTXΦ virus is meant the particle of protein-coated DNA produced by certain *V. cholerae* strains, which is capable of transducing its DNA, comprising cholera toxin genes, to other vibrios.

By cholera toxin (CT) is meant the protein responsible for the clinical symptoms of cholera when produced by the bacteria.

By CTXΦ-encoded toxin genes are meant, in addition to CT genes, zot and ace genes that encode for the "zonula occludens toxin" and for the accessory cholera enterotoxin, respectively The activity of ZOT is responsible for the destruction of the tight junctions between basolateral membranes of the epithelial cells and ACE protein has an activity accessory to that of the cholera toxin.

The term well tolerated vaccine or well tolerated strain refers to such strain lacking the residual reactogenicity that characterize most of the non-toxigenic strains of *V. cholerae.* In practical terms, it means that it is a strain safely enough to be used in communities without or with limited access to healthcare institutions without risks for the life of the vaccinees. It should be expected a rate of diarrhea in 8% or less of the vaccinees and the diarrhea is characterized in that it does not exceed 600 ml (grs), only 1% of the vaccinees or less could suffer from headache, which should be minor and of short duration (less than 6 h), and finally that it prompts vomits in less than 0.1 % of the vaccinees, those vomits characterized for being a single episode of 500 ml or less.

By hemagglutinin protease (HA/P) is meant the protein secreted by *V.* cholerae manifesting dual function, being one of them the ability to agglutinate the erythrocytes of certain species and the other the property to degrade or to process proteins such as mucine and the cholera toxin.

By *celA* is meant the nucleotide sequence coding for the synthesis of the endoglucanase A. This protein naturally occurs in *Clostridium thermocellum* strains and has a β (1-4) glucan-glucano hidrolase activity able to degrade cellulose and its derivatives.

The term MSHA is referred to the structural fimbria of the surface of *V. cholerae* with capacity to agglutinate erythrocytes of different species and that is inhibited by mannose.

By reversion to virulence mediated by VGJΦ is meant the event in which a previously attenuated strain obtained by the suppression of CTXΦ genes reacquire all the genes of this phage through a mechanism completely dependent and mediated by VGJΦ and the interaction with its receptor, MSHA.

The possibility of disseminating the CTXΦ phage in a process mediated by VGJΦ is that in which the filamentous phage VGJΦ form a stable hybrid structure (HybPΦ) through genetic recombination with the DNA of CTXΦ and disseminate its genome with active genes toward other strains of *V*. *cholerae,* which could be environmental non pathogenic strains, vaccine strains or other from different species.

### Second, information of the previous art:

Clinical cholera is an acute diarrheal disease that result from an oral infection with the bacterium *V. cholerae.* After more than 100 years of research in cholera there remains the need for an effective and safe vaccine against the illness. Since 1817 man has witnessed seven pandemics of cholera, the former six were caused by strains of the Classical biotype and the current seventh pandemic is characterized by the prevalence of strains belonging to El Tor biotype. Recently, beginning in January of 1991, this pandemic extended to South America, and caused more than 25 000 cases of cholera and over 2 000 deaths in Peru, Ecuador and Chile. By November 1992, a new serogroup of *V*. *cholerae* emerged in India and Bangladesh, the O139, showing a great epidemic potential and generating great concern through the developing world. These recent experiences reinforce the need for effective cholera vaccines against the disease caused by *V*. *cholerae* of serogroups O1 (biotype El Tor) and O139.

Because convalescence to cholera is followed by an state of immunity lasting at least three years, much efforts in *Vibrio cholerae* vaccinology have been made to produce live attenuated cholera vaccines, that closely mimics the disease in its immunization properties after oral administration, but do not result reactogenic to the individuals ingesting them (diarrhea, vomiting, fever). Vaccines of this type involve deletion mutations of all toxin genes encoded by CTXΦ. For example, the suppression of the cholera toxin and other toxins genes encoded in the prophage CTXΦ is a compulsory genetic manipulation during the construction of a live vaccine candidate (see inventions of James B. Kaper, WO 91/18979 and John Mekalanos WO 9518633 of the years 1991 and 1995, respectively).

This kind of mutants have been proposed as one dose oral vaccines, and although substantially attenuated and able to generate a solid immune responses (Kaper J. B. and Levine M. Patentes US 06,472,276 and 581,406). However, the main obstacle for the widespread use of those mutants has been the high level of adverse reactions they produce in vaccinees (Levine and cols., Infect. and lmmun. Vol 56, No1, 1988).

Therefore, achieving enough degree of attenuation is the main problem to solve during the obtainment of live effective vaccines against cholera. There are at least three live vaccine candidates, which have shown acceptable levels of safety, i.e., enough degree of attenuation and strong immunogenic potential. They are *V. cholerae* CVD103HgR (Classical Biotype, serotype Inaba) (Richie E. and cols, Vaccine 18, (2000): 2399-2410.), *V. cholerae* Perú-15 (Biotype El Tor, serotype Inaba) (Cohen M., and cols. (2002) Infection and Immunity, Vol 70, Not. 4, pag 1965 - 1970) and *V. cholerae* 638 (Biotype El tor, serotype Ogawa) (Benitez J. A. and cols, (1999), Infection and Immunity. Feb; 67(2):539-45).

Strain CVD103HgR is the active antigenic component of a live oral vaccine against cholera licensed in several countries of the world, the strains Perú-15 and 638 are other two live vaccine candidates to be evaluated in field trials in a near future.

However, there is a second problem of importance to solve in those live attenuated vaccine candidates; one is the environmental safety, specially related with the possible reacquisition and dissemination of the cholera toxin genes by existent mechanisms of horizontal transfer of genetic information among bacteria. In accordance with this, the attenuated vaccine strains of *V. cholerae,* could potentially reacquire virulence genes out of the controlled conditions of the laboratory, in an infection event with CTXΦ phage (Waldor M. K. and J. J. Mekalanos, Science 272:1910-1914) coming from other vibrios and later on contribute to their dissemination. This process could become relevant during vaccination campaigns where people ingest thousands of millions of attenuated bacteria and keep shedding similar quantities in their stools during at least 5 days. Once in the environment, bacteria have the possibility of acquiring genetic material from other bacteria of the same or different species of the ecosystem. For these reasons, at present it is desirable to obtain vaccine candidates with certain characteristics that prevent or limit the acquisition and dissemination of CTXΦ, and especially of the genes coding for the cholera enterotoxin. As a consequence, this is the field of the present invention.

Bacterial viruses, known as bacteriophages, have an extraordinary potential for gene transfer between bacteria of the same or different species. That is the case of CTXΦ phage (Waldor M. K. and J. J. Mekalanos, 1996, Science 272:1910-1914,) in *V. cholerae.* CTXΦ carries the genes that encode cholera toxin in *V*. *cholerae* and enters to the bacteria through interaction with a type IV pili, termed TCP, from toxin co-regulated pilus. TCP is exposed on the external surface of the vibrios. In accordance with published results, under optimal laboratory conditions the CTXΦ phage reaches titers of 10⁶ particles or less by ml of culture in the saturation phase; this allows classifying it as a moderately prolific bacteriophage. Equally the expression of the TCP receptor of this phage has restrictive conditions for its production. In spite of these limitations, the existence of this couple bacteriophage-receptor, limits in some way the best acceptance of live cholera vaccines, that is why depriving the bacteria from the portal of entrance to this phage is a desirable modification.

There are two theoretical ways of preventing the entrance of CTXΦ into *V. cholerae,* 1) suppressing the expression of TCP or 2) removing the TCP sites involved in phage receptor interaction. None of the two forms has been implemented due to the essentiality of TCP for proper colonization of the human intestine and elicitation of a protective immune response. It should be noted that sites involved in the TCP-CTXΦ interaction are also needed for the colonization process. (Taylor R. 2000. Molecular Microbiology, Vol (4), 896 -910).

Several strategies that counteract the entrance of the virus have been evaluated such as preventing the integration of the phage to the bacterial chromosome and its stable inheritance, consisting in the suppression of the integration site and in the inactivation of *recA* gene to avoid recombination and integration to other sites of the chromosome. (Kenner and cols.1995. J. Infect. Dis. 172:1126 - 1129).

Also, it has been recently described that the entry of CTXΦ into *V. cholerae* depends on the genes TOIQRA, however this mutation produces sensitive phenotypes not desired in vaccine candidates of cholera and it has not been implemented. (Heilpern and Waldor. 2000. J. Bact. 182:1739).

Further methods that prevent the entrance of phages carryings essential virulence determinants to cholera vaccine strains or other vaccine strains have not been described.

The main subject of the present invention is related with the phage VGJΦ and its capacity to transfer the genes coding for the cholera toxin, using the Mannose Sensitive Hemagglutinin (MSHA) fimbria as receptor. Specifically, it consists in protecting the live attenuated vaccine strains from the infection with VGJΦ by introducing suppression mutations or modifications that prevent the correct functioning of this fimbria.

In the previous knowledge of this fimbria, the following aspects can be summarized. The gene product of *mshA* was originally described to be the major subunit of a fimbrial appendage in the surface in *V. cholerae* that had the capacity to agglutinate erythrocytes of different species, this capacity being inhibited by mannose (Jonson G, and cols (1991). Microbial Pathogenesis 11:433-441). As such, the MSHA was considered a virulence factor of the bacteria (Jonson G. and cols (1994). Molecular Microbiology 13:109-118). In accordance with the attributed importance, mutants deficient in the expression of the MSHA were obtained to study its possible role in virulence. It was demonstrated that MSHA, contrary to TCP, is not required for colonization of the human small intestine by the El Tor and 0139 *V. cholerae* (Thelin KH and Taylor RK (1996). Infection and Immunity 64:2853-2856). The MSHA has been also described as the receptor of the bacteriophage 493 (Jouravleva E. and cols (1998). Infection and Immunity, Vol 66, Not 6, pag 2535-2539), suggesting that this phage could be involved in the emergence of the O139 vibrios (Jouravleva E. and cols, (1998). Microbiology 144:315-324). Later on it has been described that the fimbria MSHA has a role in biofilm formation on biotic and a-biotic surfaces contributing thus to bacteria) survival outside of the laboratory and the host (Chiavelli D. A. and cols, (2001). Appl. Environ Microbiol. Jul; 67(7):3220-25 and Watnick P. I. and Kolter R. (1999). Mol. Microbiol. Nov, 34(3):586-95). It is evident from the previous data that several investigations related with the MSHA fimbria have been done, but none of them defines this pili as the receptor of a phage able to transduce in a very efficient way the genes of the cholera toxin and not only these genes but the complete genome of CTXΦ, what could notably contribute to their dissemination. Additionally, although an extensive search has been made no inventions related with this fimbria have been found, either as virulence factor or as a phage receptor mediating dissemination of CTXΦ.

On the other hand, it is common practices among those who develop live cholera vaccines to provide them freeze-dried. Thus, these preparations of the live bacteria are ingested after the administration of an antacid solution that regulates the stomach pH and so the bacterial suspension continues toward the intestine without being damaged in the stomach and achieves colonization in the intestine.

Elaboration of freeze-dried vaccines improves preservation of strains, facilitates preparation of doses, allows a long-term storage, limits the risks of contamination and makes the commercialization and distribution easier, without the need of a cold chain, generally not available in under developing countries.

Although *Vibrio cholerae* is considered a very sensitive microorganism to the freeze-drying process, some additives are known to enhance strain survival. Thus, for preservation of the vaccine strain CVD103HgR Classical Inaba, the Center for vaccine Development, University of Maryland, United States, the Swiss Institute of Sera and Vaccines, from Beme (ISSVB), developed a formulation, see (Vaccine, 8, 577-580, 1990, S.J. Cryz Jr, M. M. Levine, J. B. Kaper, E. Fürer and B) that mainly contain sugars and amino acids. The formulation is composed of sucrose, amino acids and ascorbic acid, and after the freeze-drying process, lactose and aspartame are added.

In a work about preservation by freeze-drying of the wild type strain 569B Classical Inaba, published in Cryo-Letters, 16, 91-101 (1995) for Thin H., T. Moreira, L. Luis, H. Garcia, T.K. Martino and A. Moreno, compared the effect of different additives on the viability and final appearance upon liophilization and after the storage at different temperatures of this *V. cholerae* strain. It was demonstrated that viability losses were less than 1 logarithmic order after 3 days of storage to 45° C.

The invention CU 22 847 claims a liophilization method where the formulations contain a combination of purified proteins or skim milk with addition of polymers and/or glycine, besides bacteriologic peptone or casein hydrolysate and sorbitol, with good results for the viability of *Vibrio cholerae* strains of different serogroups, biotypes and serotypes. The freeze-dried bacteria keep their viability after being dissolved in a 1,33% sodium bicarbonate buffering solution used to regulate the pH of the stomach.

Any vaccine formulation of cholera that it is supposed to be used in under developing countries should have certain requisites such as possessing a simple composition, be easy to prepare and manipulate, be easy to dissolve and have good appearance after dissolved. Besides, it would be also desirable not to require low storage temperatures and to tolerate high storage temperatures at least for short periods of time, as well as the incidental presence of oxygen and humidity in the container. Additionally, it is also necessary an adequate selection of the composition of the formulation that allows the preservation of *Vibrio cholerae* of different serogroups, biotypes and serotypes. Finally, it is also remarkable that a formulation free of bovine derivate ingredients allows us to be in agreement with the international regulatory authorities related to the use of bovine components due to the Bovine Spongiform Encephalopathy Syndrome.

### Description of the invention.

The present invention propose a new generation of live attenuated vaccines to immunize against cholera by modification of their properties, specifically improving their biological safety during colonization of humans and later in the environment, outside the laboratories.

The present invention born from the necessity to protect live cholera vaccines from infection with the CTXΦ bacteriophage, which contains the cholera toxin genes, and also to impair the potential dissemination of this phage starting from live cholera vaccine candidates. Specifically it was born from the discovery and characterization of the VGJΦ phage in our laboratory.

VGJΦ is a filamentous bacteriophage isolated from *V. cholerae* O139 but it has infective capacity on *V. cholerae* O1 of all serotypes and biotypes and also over other strains of *V. cholerae* O139. The sequence of this phage was not described in the complete genome sequence of *V. cholerae,* indicating that this phage was not present in the strain N16961 (O1, El Tor Inaba). From a broad list of *V. cholerae* O1 strains existing in our laboratory, none of them had homologous sequences to VGJ4Φ, while strains MO45, SG25-1 and MDO12C, of *V. cholerae* O139 had.

The VGJΦ phage infects *V. cholerae* through the MSHA fimbria. When this phage enters the bacterium it can replicate or integrate into a specific chromosomal region. This is a very active phage that reaches 10¹¹ particles ml⁻¹ in the culture supernatants.

In the document Ehara. M. et al. Characterization of filamentous phages of Vibrio cholerae 0139 and 01. FEMS Microbiology letters. 1997 Vol 154 page 239-301 it is described the characterization of filamentous phages in 01 and 0139 of Vibrio cholerae, including the phage fs1, which is 96% similar in its first 1712 nucleotides to VGJΦ (SEQ ID NO 1), however its receptor is VCF in place of MSHA.

The most important characteristic in this phage, by virtue of which the following application of invention is issued, is their capacity to carry out a specialized transduction of the CTXΦ phage and consequently of the cholera toxin genes. This process occurs by a site-specific recombination between CTXΦ and VGJΦ genome, followed by the encapsulation and exportation of both genomes into the VGJΦ capsid. This hybrid viral particle was named HybPΦ. A culture of bacteria infected with both, CTXΦ and VGJΦ, produce 10¹¹ particles ml⁻¹ of VGJΦ and 10⁷-10⁸ particles ml⁻¹ of HybPΦ, which is at least 100 times higher than the titers obtained with CTXΦ alone.

It is also important to understand, to the purpose of this application that the CTXΦ phage receptor is TCP, which require special conditions for its expression, while the VGJΦ receptor is MSHA fimbria, an antigen that is expressed abundantly in all culture conditions studied and that is also produced in the environment. Furthermore, other vibrios produce the MSHA what increase the risk of transmission, even to other bacterial species.

It is also important to know that once, a new host become infected with HybPΦ, a stable production of particles in the range of 10⁷-10⁸ ml⁻¹ takes place in the saturation phase, thus this hybrid phage has a high potential to transmit and disseminate the cholera toxin genes.

Another aspect of supreme interest to the purpose of this invention is that cholera toxin genes in HybPΦ are active enough to produce 50 ng ml⁻¹ of toxin during in vitro culture and that the infection of an attenuated strain with HybPΦ revert it back to virulence as assessed by the infant mouse cholera model.

In accordance with these data, a primary objective of the present invention is to describe the additional mutations made to live cholera vaccines to prevent them to be infected with either VGJΦ or HybPΦ, as well as the necessity to use live cholera vaccines from which the genome of VGJΦ is absent to avoid the dissemination of CTXΦ mediated by VGJΦ, in the case of reacquisition of CTXΦ.

An example of this mutation is a stable spontaneous mutation, conducive to the lack of expression of the MSHA fimbria in the cellular surface. This way, the VGJΦ or its derivative phage, HybPΦ could not infect such vaccines.

Another example of this mutation is a suppressive mutation in the structural gene of the major protein subunit of this fimbria (MshA).

The use of live cholera vaccine candidates in which the genome of VGJΦ is absent could be achieved simply by searching for hybridization of DNA in different strains to identify which have not homologous fragments to VGJΦ described in this invention, although other well known methodologies could be applied to remove VGJΦ from an infected strain.

Examples of live cholera vaccines to perform the specific mutations mentioned above are vaccine strains which are not able to react with a VGJΦ specific probe and that have been demonstrated in the previous art, to have acceptable levels of reactogenicity in volunteers studies. The genotype of these strains includes suppressive mutations of CTXΦ phage leaving a remnant RS1 and the insertional inactivation of *hap* gene with the *cel*A gene. Such strains are constructed by means of traditional methods of suppression of the CTXΦ prophage in epidemics strains of *V*. *cholerae,* followed by the inactivation of the hemagglutinin protease gene (*hap*) for the insert of the marker gene *celA* in their sequence. To see Robert's scientific article and cols., Vaccine, vol 14 No16, 1517-22, 1996), the scientific article of Benitez J. A. and cols, (1999), Infection and Immunity. Feb; 67(2): 539-45, and the application of invention WO9935271A3, of Campos and cols, 1997. Other strains with these characteristic and that additionally have auxotrophy mutations are also useful to obtain the strains with the characteristics of interest of the present invention.

In accordance with the description in the above paragraph, a primary objective of this invention is to protect the use of suppressive or spontaneous mutations conductive to the absence of the MSHA fimbria in the surface of vibrios and in this way impede that live cholera vaccine reacquire and disseminate the cholera toxin genes by means of the infection with the hybrid phage, HybPΦ.

Among the preferred inclusions of this invention are any live cholera vaccine strain of the existing biotypes and serotypes or any non toxigenic strain of another emergent serotypes with genetic manipulations that suppress the genome of the CTXΦ phage, inactivate the hap gene, combined with any other mutation, for example the introduction of some auxotrophies (to lysine or metionine) and that also have the characteristics proposed in the present invention.

Among the preferred inclusions are also the use of the well tolerated live cholera vaccines, improved by the impossibility of acquiring CTXΦ in an event mediated (by) VGJΦ and for the absence of VGJΦ that diminish the risk of dispersion of CTXΦ, as a delivery system to present heterologous antigens to the mucosal immune system.

To obtain these mutants in the expression of the MSHA fimbria we have used several molecular biology techniques which are not object of protection of the present document.

The present invention also discloses the methods to preserve and lyophilizate these strains with the purpose of being able to prepare live vaccines that present a rapid and adequate reconstitution post lyophilization without affecting their viability when being reconstituted in a solution of sodium bicarbonate 1,33%.

It is also the object of the invention that by means of the adequate selection of components, the lyophilized formulations guarantee that live cholera vaccines does not decrease their viability less than 1 logarithmic order as consequence of the storage, independently of the serogroup, serotype or biotype or the mutations they have, even if they were lyophilized for separate or mixed as part of a same preparation.

Among the formulation components to be present are lactose (L), peptone (P), yeast extract (E) and sorbitol (S). The total concentration should not exceed the 10%.

### Example 1: Discovery and characteristics of the VGJΦ phage

VGJΦ was discovered as an extrachromosomal transmissible element in total DNA preparations from *Vibrio cholerae* SG25-1, an O139 strain isolated in Calcuta India, 1993 and kindly donated by professor Richard A. Finkelstein. Simple experiments showed the transmissibility of this element. Free-cell culture supernatants of the donor strain, carrying the element, was grown in standard condition like LB media (NaCl 10 g/l, triptone 10 g/l and yeast extract 5 g/l) and was able to transfer to a receptor strain that does not contain any extrachromosomal element, one genetic element of the same size and restriction map that the one was present in the donor strain. The property of transmission without the direct contact between donor-receptor is typical in phages.

Infection assays: Donor strains were grown until optical density to 600nm equal 0.2. One aliquot from the culture was filtered through a 0.22 µm- pore-size filter to remove the bacteria. The sterility of the filtrate was confirmed by growing one aliquot in LB plates and incubating overnight at 37°C. After checking the lack of colony forming units, 100 µl of free-cell supernatants or serial dilutions were used to infect 20 µl of a fresh culture of the receptor strain. The mixture was incubated for 20 min at room temperature and spread in solid or liquid LB media at 37 °C overnight. The infection was confirmed by the presence of replicative form (FR) and single strand DNA (ssDNA) of VGJΦ in the infected vibrios.

Purification of VGJΦ phage: Purification of phage particles was done from 100 ml of the culture of 569B *Vibrio cholerae* strain (classic, Inaba) infected with VGJΦ. This strain was used because it contains a CTXΦ defective prophage. The cells were centrifuged at 8000 x g for 10 min. The supernatant was filtered through a 0.22 µm membrane. Phage particles in the filtrate were precipitated by addition of NaCI and polyethylene glycol 6000 to a final concentration of 3 and 5 % respectively. The mixture was incubated in ice for 30 min and centrifuged at 12000 x g for 20 min. The supernatant was discarded, and the phage-containing pellet was suspended in 1 ml of phosphate buffered saline.

Characterization of VGJΦ: VGJΦ particles precipitated retained the capacity to infect 569B strain and were stable in PBS solution during at least 6 month at 4°C.

After phage particles purification, the genomic DNA was extract using phenol-chloroform solution. The analysis of this DNA showed resistance to digestion with ribonuclease H indicating that the genome is DNA and not RNA (data not shown) and it was also resistant to the treatment with different restriction enzyme but sensitive to treatment with Mung-Bean and S1 nuclease (data not shown), indicating that the phage genome consists of ssDNA. An electrophoresis analysis in the presence of acrydine orange demonstrated similar results to the previous ones. The acrydine orange intercalated in the double stranded DNA (dsDNA) fluoresce green, while fluoresce orange when intercalates in the ssDNA. As expected, the genomic DNA fluoresced orange indicating its single stranded nature (data not shown) and the plasmid DNA observed in the infected cells fluoresced green indicating that it consists of dsDNA.

Identity between the genome of VGJΦand the intracellular replicative form. Southern blotting analysis carried out using the genome of VGJΦ as a probe showed a genetic identity between the extrachromosomal elements of the donor strain SG25-1 and the infected strain 569B. This result confirms that the ssDNA of the viral genome is produced by the cytoplasmic RF and at the same time suggests that VGJΦ is a filamentous phage, which uses the rolling circle mechanism of replication to produce the genomic ssDNA that is assembled and exported in phage particles.

The RF, isolated from the infected strain 569B, was mapped by restriction analysis. The map obtained showed that the phage genome size (about 7500 b) and the electrophoretic restriction pattern were different to those of the previously reported *V.* cholerae-specific filamentous phages. These results indicated that the phage isolated from SG25-1 was not described previously and it was designated VGJΦ.

Titration of VGJΦ. For tittering the phage suspensions the procedure was the same as the infection assay, but the indicator strain cells were plated onto an overlay of soft agar (0,4%) over solid LB plates. The plates were incubated overnight at 37°C and the observed opaque plaques (infection focuses) were counted.

This assay revealed that a culture of 569B infected with VGJΦ is able to produce until 3x10¹¹ phage particles per ml of culture, what is unusually high compared with other described filamentous phages of *V. cholerae* like CTXΦ, which produces a maximum of 10⁶ particles per ml.

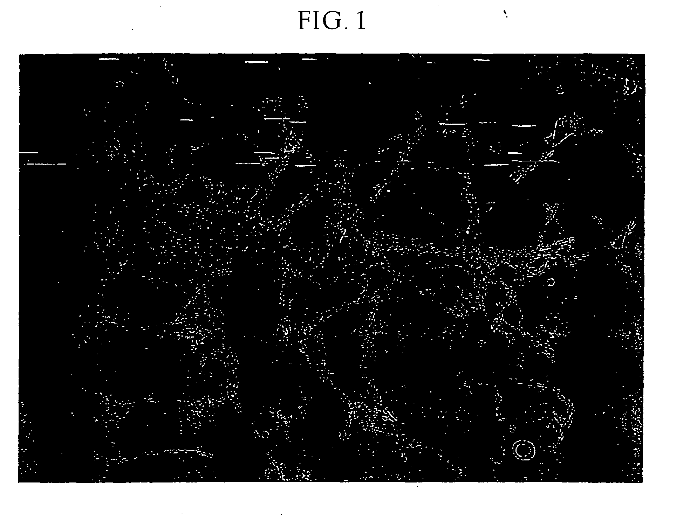

Electron microscopy. Different quantities of VGJΦ particles were negatively stained with a solution of 4% uranile acetate (m/v) and observed over a freshly prepared Formvar grids in a transmission electron microscope JEM 200EX (JEOL, Japan). The observation confirmed that the phage particles had a filamentous shape (Fig. 1).

Construction and titration of VGJ-KnΦ. The RF of VGJΦ was linearized by its unique Xbal site. One DNA fragment containing the R6K replication origin and a kanamycin resistance cassette from pUC4K plasmid was inserted in the Xbal site of VGJΦ. This recombinant RF was introduced in *V. cholerae* 569B and the phage particles were designated as VG.J-KnΦ.

The donor strain, 569B infected with VGJ-KnΦ, was cultured until an OD₆₀₀=2.0. An aliquot of the culture was filtered through a 0.2 um-pore-size filter to eliminate the bacterial cells. The sterility of the cell-free suspension was checked by plating an aliquot of 50 ul in a solid LB plate and incubating overnight at 37°C. Aliquots of 100 ul of the cell-free phage suspension or dilutions of it were used to infect 20 µl of a fresh culture of the receptor strain (about 10⁸ cells). The mixture was incubated at RT for 20 min to allow infection. Subsequently, the mixtures were plated onto solid LB supplemented with kanamycin (50 µg/ml) and the plates were incubated overnight at 37°C. The colonies that grow in the presence of antibiotic acquired their Kn-resistance due to the infection with the marked phage VGJ-KnΦ. Several of these colonies were checked for the presence of the RF of VGJ-knΦ by purification of plasmid DNA and restriction analysis of it.

Titration assay done by this method agreed with those obtained by that of opaque plaques with VGJΦ, showing that a culture of 569B infected by VGJ-knΦ produces about 2 x 10¹¹ particles of phage VGJ-knΦ per milliter of culture.

Nucleotide sequence: The nucleotide sequence of VGJΦ consisted of 7542 nucleotides and had a G+C content of 43.39%. The codified ORFs were identified and compared to protein data bases.

The genomic organization of VGJΦ was similar to that of previously characterized filamentous phage, such as phages of Ff group (M13, fd and f1) of *E*. *coli* and other filamentous phages of *V. cholerae* (CTXΦ, fs1, fs2 and VSK) and *V. parahemolyticus* (Vf12, Vf33 and VfO3k6). VGJΦ does not have a homologous gene to the gene IV of phages of Ff group which suggests that VGJΦ could use a porine of the host for assembling and exporting its phage particles, similar to CTXΦ phage.

The nucleotide sequence of VGJΦ revealed that VGJΦ is a close relative of fs1 and VSK phages, sharing several ORF highly homologous and exhibiting 82.8 and 77.8% of DNA homology to VSK and fs1. However, there are genome areas highly divergent and ORFs not share between them. Besides, the genome size is different and it has not been described before that fs1 or VSK being capable of transducing the genes of cholera toxin.

The nucleotide sequence of VGJΦ also revealed the presence of two sites homologous to att sequences known to function in integrative filamentous phage. These sites of VGJΦ are partially overlapped and in opposite directions. This arrangement was also found in phages Cf1c, Cf16-v1 and (ΦLF of *X*. *campestris* as well as Vf33 and VfO3k6 of *V. parahemolyticus* and VSK of *V. cholerae.* All these phages except Vf33 and VSK integrate in the chromosome of their hosts by the att site present in the negative strand of the replicative form of these phages.

### Example 2. Identification of VGJΦ receptor.

Filamentous phages generally use type IV pili as receptor to infect their hosts. Previously reported *V. cholerae*-specific filamentous phages use TCP or MSHA pili as receptor. Therefore, two mutants of the El Tor strain C6706 for these pili, KHT52 (Δ*tcpA*10) and KHT46 (Δ*mshA*), were used to identify if any of them was the receptor of VGJΦ. While parenteral strain C6706 and its TCP-mutant KHT52 were sensitive to the infection with VGJΦ, the MSHA-mutant KHT46 was fully resistant to the phage, indicating that MSHA was the receptor of VGJΦ. Complementation of strain KHT46 with wild type *mshA* structural gene (from parental C6706) carried on plasmid pJM132 restored phage sensitivity, confirming that MSHA is the receptor for VGJΦ. The resistance or sensitivity to VGJΦ was evaluated by the absence or presence of replicative form in cultures of receptor strain analyzed after the infection assay.

To give a numerical titer of the particles which are transduced in each case, it was used an infection assay with VGJΦ-kn as was described previously, resulting the following:

The parental strain C6706 and its derivative TCP mutant KHT52 were sensitive to the infection with VGJΦ-Kn and, as indicator strains showed titres of 10¹¹ plaque forming units (PFU), while KHT46, a MSHA mutant, was fully resistant to the phage, less than 5 PFU/mL, after being infected with the same preparation of VGJΦ-Kn. Complementation of strain KHT46 with wild type *mshA* structural gene, restored phage sensitivity. These results confirm that a mutation that prevents the expression of MSHA pilus confers resistance to the VJGΦ infection.

Further assays to compare the capacity of HybPΦ and CTXΦ to infect Clasical and El Tor strains were done, using their kanamycin resistant variants. See the results in Table 1.

As it has been previously described, CTXΦ-Kn phage was obtained through the insertion of a kanamycin resistance cassette from the plasmid pUC4K (Amersham Biosciences), in the unique restriction site, Notl, of the replicative form of CTXCIΦ.

The HypPΦ phage was obtained during an infection assay where cell free culture supernatant of 569b strain co-infected with CTXΦ-Kn and VGΦ-Kn was used to infect the receptor strain KHT52. The cells of this strain carrying kanamycin resistance, originally carried by CTXΦ-Kn and provided to HybPΦ, were purified and, they continued producing HybPΦ viral particles to the supernatant.

To check the efficiency of infection of the hybrid phage in Classical and EI Tor vibrios, suspensions of CTXΦ-Kn and VGJΦ-Kn of the same title (1-5x10⁵ particles / ml) were used to infect the receptor strains 569B (Classical) and C7258 (El Tor). In both cases, the receptor strains were grown in optimal condition for TCP expression, the CTXΦ receptor. The assay was done as follows, 200 µL of pure phage preparation were mix with 20µL (about 10⁸ cells) of a fresh culture of a receptor strain during 20 min at room temperature, plated on solid LB supplemented with kanamycin and incubated over nigh at room temperature.
The numbers of colonies carry the Kn-resistance gene in their genome is the result of phage infections and show the capacity of each phage to infect different strains in routine laboratory condition. Those results are exposed in Table 1.

**Table 1. Titration of CTXΦ-Kn and hybrid (HybPΦ-Kn) phages in 569B and C7258.**

| **Phage** | **Kn^{r} colony number of receptor strain** | |
|---|---|---|
| | **569B** | **C7258** |
| CTXΦ-Kn | 5.8 x 10⁵ | 0 |
| HybPΦ-kn | 1.5 x 10⁵ | 7.5 x 10⁴ |

As it is shown in Table 1 the hybrid phage transduces CT genes more efficiently than CTXΦ, the ordinary vehicle of these genes. These results point out the importance of the CTXΦ transmission mediated by VGJΦ among *Vibrio cholerae* strains and stressed its relevance considering the ubiquity of MSHA, the functional receptor in these bacterial strains.

### Example 3. Mobilization of CTXΦ, its mechanism and reversion to virulence.

Infection of *V*. *cholerae* O1 or O139 strains that carry an active CTXΦ phage with VGJΦgives rise to the production of infective particles that bear the CTXΦ phage genome inserted in the genome of VGJΦ. These particles of hybrid phages have been designated HybPΦ. The HypΦ titers were evaluated by means of the use of a hybrid phage, which carries a kanamycin marker (HybPΦ-Kn), employing different strains as indicators. The resultant titers are shown in Table 1.

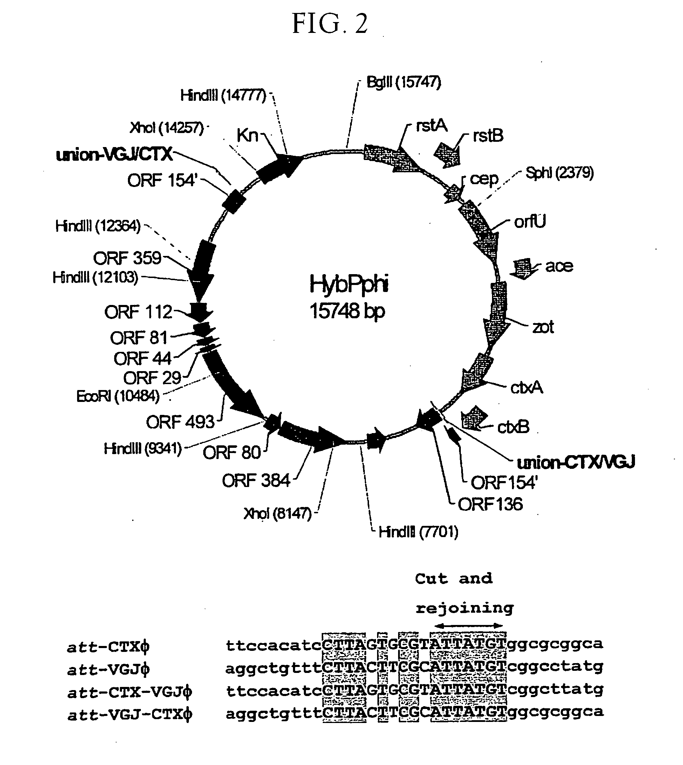

HybPΦ-Kn was purified starting from preparations derived of 569B (HybPΦ-Kn) strain and the single strand was sequenced to determine the junctions between CTXΦ and VGJΦ. The cointegrate structure is graphically shown in the Figure 2 and the nucleotide sequences of the junctions among both sequences, what explains the mechanism by which VGJΦ transduces CTXΦ toward other *V. cholerae* strains. HybPΦ-Kn enters to *V. cholerae* using the same receptor that VGJΦ, that is to say MSHA.

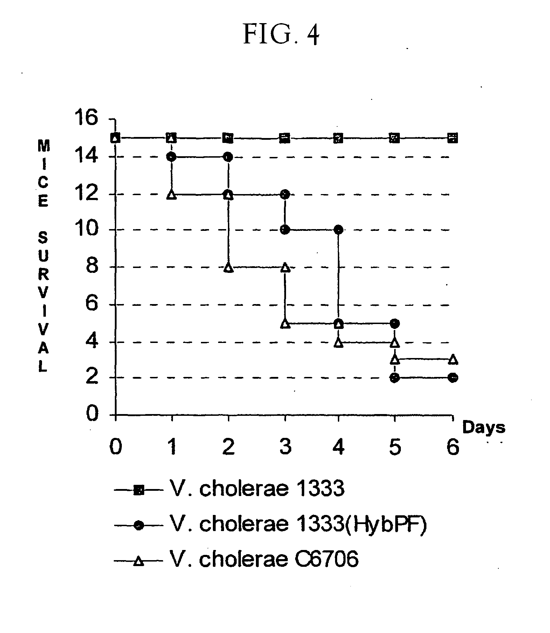

*V. cholerae* 1333 strain is an attenuated clone described in the previous art, similar to the strains that were useful for obtaining the derivative of the present invention. This strain is a derivative of the pathogenic C6706 strain. As it shows in the Figure 4, the inoculation of 10⁵ colony-forming units of 1333 strain in suckling mouse does not have lethal effect, even when it is colonizing for the subsequent 15 days. Several experiments to determine virulence, demonstrated the effect of the HybPΦ-Kn infection on the reversion to virulence. While a dose of 10⁵ CFU of 1333 strain does not have a lethal effect, C6706 and 1333 (HybPΦ-Kn) strains have very similar lethality profiles, and don't allow survival of inoculated mice beyond the fifth day (Figure 4).

### Example 4. Constitutive expression of the VGJΦ receptor, the MSHA fimbria, in different culture conditions.

To study the expression of MshA, the major subunit of MSHA fimbria, *V. cholerae* C7258, C6706, and CA401 strains, were grown in different media. The media used were: LB pH 6.5 (NaCl, 10 g/l; bacteriological triptone, 10 g/l; yeast extract, 5 g/l), AKI (bacteriological peptone, 15 g/l; yeast extract, 4 g/l; NaCl, 0.5 g/l; NaHCO3, 3 g/l), TSB (pancreatic digestion of casein, 17 g/l; papaine digestion of soy seed, 3.0 g/l; NaCl, 5 g/l; dibasic phosphate of potassium, 2.5 g/l; glucose, 2.5 g/l), Dulbecco's (glucose, 4.5 g/l; HEPES, 25 mm; pyridoxine, HCI, HaHCO3), Protein Free Hybridoma Médium (synthetic formulation free of serum and proteins, suplemented with NaHCO3, 2.2 g/l; glutamine, 5 mg/); red phenol, 20 g/l) and Syncase (NaH2PO4, 5 g/l; KH2PO4, 5 g/l; casaminoacids, 10 g/l; sucrose, 5 g/l and NH4CI, 1.18 g/l). In all cases was inoculated one colony in 50 ml of culture broth and was grown in a rotary shaker during 16 hours at 37°C, with the exception of the AKI condition in which the strains were grown first at 30°C in static form during 4 hours and later on rotator shaker at 37°C during 16 hours. In each case, the bacterial biomass were harvested by centrifugation and used to prepare cellular lisates. Equivalent quantities of cellular lisates were analyzed by Western Blot with the monoclonal antibody 2F12F1 for immunodetection of mshA. The MSHA mutant strain KHT46 was used as negative control of the experiment. All the studied strains, except the KHT46 negative control strain, showed capacity to produce MshA in all culture conditions tested. Equally, said strains cultured in the previous conditions have the capacity to hemagglutinate chicken erythrocytes (mannose sensitive), in the same titer or higher to 1:16 and are efficiently infected by VGJΦ-Kn, exhibiting titers higher than 10¹⁰ particles per milliliter of culture.

### Example 5. Obtaining of spontaneous mutants deficient in MSHA expression and evaluation of resistance to infection

Strain KHT46, a MSHA suppression mutant, derived from *V. cholerae* C6706 (O1, The Tor, Inaba), shows a refractory state to the infection with VGJΦ, VGJΦ-Kn and the hybrid HybPΦ phages. However, this is a pathogenic strain that is not property of the authors of the present application, neither of the juridical person who presented it, The National Center for Scientific Research, in Havana City, Cuba.

To obtain the spontaneous mutants deficient in the expression of superficial MSHA of the present application, was used a suppression mutant in the cholera toxin genes that during the process of obtainment resulted affected in their capacity to assemble MSHA in the cellular surface. Said mutants although are capable of producing the structural subunit of MSHA, do not assemble it in their surface and therefore do not have detectable titers of mannose sensitive hemagglutination, neither adsorb the activity of a specific monoclonal antibody against the MSHA in a competition ELISA. Since this phenotype is notably stable, these mutants were subsequently genetically manipulated to introduce an insertional mutation in the hemagglutinin protease gene, following the procedure described in patent WO 99/35271 *"V. cholerae* vaccine candidates and the methods of their constructing" of Campos *et al,* and in the Robert's article, Vaccine, vol 14 No 16, 1517-22, 1996. The resultant mutants were named JCG01 and JCG02, both of O1 serogrup, El Tor biotype, Ogawa serotype.

JCG01 and JCG02 showed a refractory state to the infection with the VGJΦ-Kn phage, a variant of the VGJΦ phage that carries a resistance marker to kanamycin. A VGJΦ-Kn suspension that had a proven titer of ~ 10¹¹ units per ml, does not show capacity to infect said strains (non detectable titers, lower to 5 units for ml). This refractory state to the infection with VGJΦ-Kn correspond with a very low titer of hemagglutination in the strains JCG01 and JCG02 (1:2) regarding their parental (1:32) besides a total impairment in the MSHA dependent hemaglutination. Equally, whole cells of these mutants had null capacity to inhibit the interaction of the anti-MSHA monoclonal antibody (2F12F1) to MshA fixed on the solid phase in a competition ELISA. However, both strains produced the major structural subunit MshA, according to immunoblot experiments, indicating that the protein is not correctly assembling in the cellular surface although it is being produced. These mutants allowed proving the concept of this invention and passing to obtain suppression mutants.

Obtaining suppression mutants in the *mshA* gene starting from other cholera vaccine candidates. To obtain suppression mutants in the *mshA* structural gene, two segments of the genome of *V. cholerae* N16961, of - 1200 base pairs for each flank of the *mshA* structural gene were amplified by means of the polimerase chain reaction, using the following oligonucleotides: CNC-8125, ATG ATC GTG AAG TCG ACT ATG (21 mer); CNC-8126 CAG CAA CCG AGA ATT HERE ATC ACC ACG (27 mer); CNC-8127, ATT CTC GGT TGC TGG AAC TGC TTG TG (26 mer); and CNC-8128, GCT CTA GAG TAT TCA CGG TAT TCG (24 mer). The amplified fragments were cloned independently and assembled in vitro to generate the pΔmshA clone. This clone contains these fragments in the same order and orientation that they are found in the bacterial chromosome; only the coding region of the *mshA* gene has been suppressed from the inner of the sequence. The fragment carrying the suppression was subcloned from the previous plasmid as a *Sal* I/Xba I fragment in the suicide vector pCVD442 to obtain the plasmid pSΔmshA.

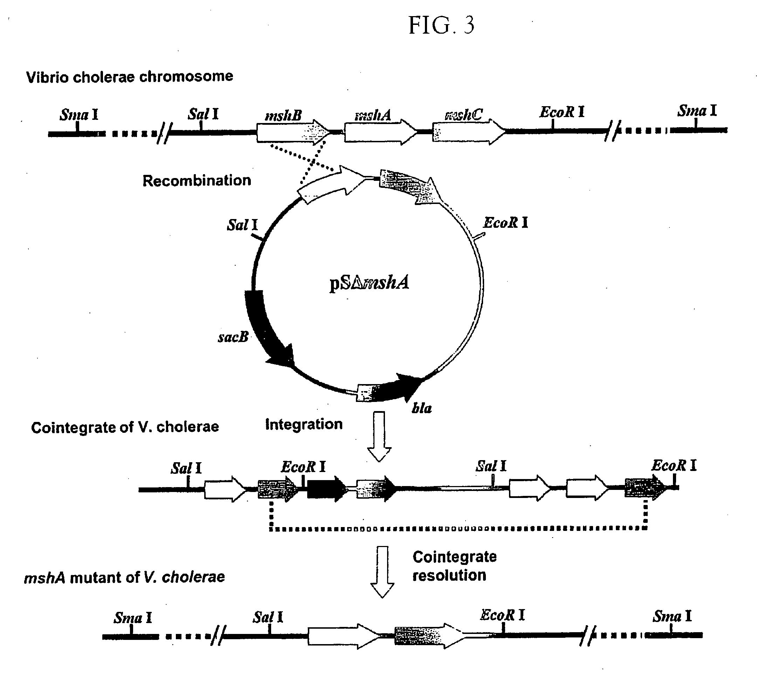

The plasmid pSΔmshA was used to suppress the chromosomal *mshA* gene in the *V. cholerae* vaccine strains by means of a traditional methodology of allelic replacement. For it, pSΔmshA was introduced in the *E*. *coli* strain SM 10lpir and mobilized toward *V*. *cholerae* by means of a procedure of bacterial conjugation. The resultant clones were selected for their resistance to the ampicillin antibiotic in plates of LB medium supplemented with ampicillin (100 mg/ml). Most of these clones arise due to integration of the plasmid in the chromosome of the receptor vibrios by means of an event of homologue recombination between one of the flanking fragments to the chromosome *mshA* gene and that of the plasmid pSΔmshA, originating a cointegrate between both. This event was verified by means of a Southern blot experiment, in which the total DNA of 10 clones was digested with the restriction enzyme *Sma* I and hybridized with a probe obtained from the plasmid pSΔmshA (*Sal* I/Xba I insert). The clones of our interest are those that produce a band of 21 000 base pairs. A similar control of the parental strain in this experiment produced a band of 13 000 base pairs. The adequate clones were conserved immediately in LB glycerol at -70°C. Then 3 of them were cultured in the absence of the antibiotic selective pressure to allow that an event of homologue recombination eliminated the genetic duplication existing. This can happen by means of suppression of the original genetic structure (intact *mshA* gene) and replacement by a mutated copy present in the plasmid (supressed *mshA* gene) as is shown in Figure 3. The clones in which the mutated gene replaced the intact gene were analyzed by Southern blot and identified by the presence of a band of 12 000 base pairs. Finally, the clones where the *mshA* gene was suppressed were selected and conserved appropriately as vaccine candidates (freezing at -80°C in LB supplemented with 20% glycerol). This procedure was performed with each clone where the *mshA* suppression mutant was constructed.

Serological characterization. After the introduction of each mutation in the vaccine strains described in this document, each derivative was checked for the correct expression of the lipopolysaccharide corresponding to the original serotype. For that, cells were collected from a fresh plate, resuspended in saline (NaCI, 0.9%) and immediately examined with an appropriate agglutination serum, specific for Ogawa, Inaba or 0139 vibrios.

The major immune response generated by an anti-cholera vaccine, is against the LPS, therefore the expression of the antigen corresponding to each one of the strains presented in this invention was confirmed by agglutination with specific antiserum.

Colonization assay in suckling mice. The colonization assay in suckling mice (Herrington et al., J. Exper. Med. 168: 1487-1492, 1988) was used to determine the colonizing ability of each strain. An inoculum of 10⁵-10⁶ vibrios in a volume of 50 ml was administered by orogastric route to groups of at least 5 suckling mice. After 18-24 hours at 30°C the mice were sacrificed, the intestine was extracted and homogenized, and dilutions were plated in appropriate media for the growth of mutants.

**Table 2. Colonizing capacity of the vaccine strains of the present invention.**

| Strain | Inoculum | Colonizing | Genotype |
|---|---|---|---|
| BLR01 | 1.0 x 10⁵ | 2.8 x 10⁴ | ΔCTXΦ, *hap*::*celA,* Δ*mshA* |
| BLR02 | 2.0 x 10⁶ | 4.2 x 10⁴ | ΔVGJΦXΦ, *hap*::*celA, lysA,* Δ*mshA* |
| BLR03 | 1.2 x 10⁶ | 8.0 x 10³ | ΔCTXΦ, *hap*::*celA, metF,* Δ*mshA* |
| EMG01 | 3.0 x 10⁵ | 8.0 x 10⁶ | ΔCTXΦ, *hap*::*celA,* Δ*mshA* |
| EMG02 | 2.5 x 10⁵ | 3.0 x 10⁶ | ΔVGJΦXΦ, *hap*::*celA, lysA,* Δ*mshA* |
| EMG03 | 4.0 x 10⁵ | 5.0 x 10⁵ | ΔCTXΦ, *hap*::*celA, mefF,* Δ*mshA* |
| JCG01 | 2.0 x 10⁵ | 6.0 x 10⁶ | ΔCTXΦ, *hap*::*celA,* MSHA⁻ |
| JCG02 | 1.0x10⁵ | 6.0 x 10⁷ | ΔCTXΦ, *hap*::*celA,* MSHA⁻ |
| JCG03 | 1.0 x 10⁵ | 1.0 x 10⁶ | ΔCTXΦ, *hap*::*celA,* Δ*mshA* |
| EVD01 | 3.0 x 10⁵ | 3.0 x 10⁵ | ΔVGJΦX<Φ, *hap*::*celA, thyA,* Δ*mshA* |
| KMD01 | 1.0 x 10⁶ | 7.0 x 10⁵ | ΔCTXΦ, *hap*::*celA, metF,* Δ*mshA* |
| KMD02 | 2.0 x 10⁶ | 5.0 x 10⁶ | ΔCTXΦ, *hap*::*celA, lysA,* Δ*mshA* |
| ESP06 | 1.7 x10⁶ | 6.0 x10⁵ | ΔCTXΦ, *hap*::*celA,* Δ*VC0934,* |
| JCG04 | 1.0 x 10⁶ | 2.0x10⁷ | ΔCTXΦ, *hap*::*celA,* Δ*mshA* |
| ESP01 | 1.0 x 10⁵ | 5.0 x 10⁶ | ΔVGJΦXΦ, *hap*::*celA, metF,* Δ*mshA* |
| ESP02 | 6.0 x 10⁶ | 4.0 x 10⁵ | ΔCTXΦ, *hap*::*celA, lysA,* Δ*mshA* |
| ESP04 | 8.0 x 10⁴ | 1.0 x 10⁶ | ΔCTXΦ, *hap*::*celA,* Δ*VC0934,* |
| RAF01 | 3.1 x 10⁵ | 5.0 x 10⁷ | ΔCTXΦ, *hap*::*celA, ΔmshA* |
| EVD02 | 2.8 x 10⁵ | 3.1 x 10⁶ | ΔVGJΦXΦ, *hap*::*celA, thyA,* Δ*mshA* |
| ESP03 | 1.5 x 10⁵ | 2.0 x 10⁶ | ΔCTXΦ, hap::celA, *metF,* Δ*mshA* |
| KMD03 | 2.3 x 10⁵ | 3.4 x 10⁶ | ΔCTXΦ, *hap*::*celA, lysA,* Δ*mshA* |
| ESP05 | 2.1 x 10⁶ | 2.3 x 10⁵ | ΔCTXΦ, *hap*::*celA,* ΔVC0934, |
| TLP01 | 2.3 x 10⁶ | 3.2 x 10⁵ | ΔCTXΦ, *hap*::*celA,* Δ*mshA* |
| TLP02 | 3.4 x 105 | 9.4 x 10⁴ | ΔVGJΦXΦ, *hap*::*celA, lysA,* Δ*mshA* |
| TLP03 | 2.7 x 10⁵ | 8.8 x 10⁴ | ΔCTXΦ), *hap*::*celA, metF,* Δ*mshA* |

All the strains showed adequate colonizing capacity to be used as live vaccine candidates. The colonization is needed to generate a strong immunological response because the local multiplication of the bacteria increases the duration of interaction with the mucosal immune system. In this case, although a perfect model for cholera does not exist, the suckling mice gives an adequate approach to what can be the subsequent colonization of each strain in humans.

Motility assay. The cells of a well isolated colony are loaded in the tip of a platinum loop from a master plate toward a plate for the motility detection (LB, agar 0.4%), introducing the tip of the loop 2-3 mm in the agar. The diameter of dispersion of each colony in the soft agar to 30°C is measured at 24 hours of incubation. A bacterial strain that reaches a diameter of 3 mm or less from the point of inoculation is considered as non-motile. A bacterial strain that grows in a diameter beyond 3 mm is considered as motile. All the strains included in this invention resulted to be motile.

### Example 6. Methods to select and construct the vaccine candidates useful as starting strains to be modified by the procedure disclosed in the present invention.

Five pathogenic strains in our collection were selected as starting microorganism due to their lack of hybridization with VGJΦsequences. These strains are *V. cholerae* C7258 (O1, El Tor, Ogawa, Peru, 1991), C6706 (O1, El Tor, Inaba, Peru, 1991), CRC266 (O139, La India, 1999), CA385 (Clásico, Ogawa) y CA401 (Clásico, Inaba).

The procedures disclosed in this example are not the subject of the present invention. They rather constitute a detailed description of the methods used to obtain attenuated strains that are the substrate to construct the mutants claimed in the present invention. These mutants being characterized in that they are refractory to infection by VGJΦ and the hybrid VGJΦ::CTXΦ are obtained by the methods described in the examples 4 and 5.

Below we describe the suicide plasmids used to introduce different sets of mutations into *V. cholerae* by allelic replacement before they are suitable to be modified by the methods of the present invention. The reader should note that the strains claimed in the present invention have in addition to the mutation that impairs the correct expression of MSHA fimbriae (a) a deletion mutation of the cholera enterotoxin genes or the entire CTXΦ prophage and (b) the hemaglutinin protease gene interrupted with the *Clostridium thermocellum* endoglucanase A gene. They can also have additionally and optionally mutations in the genes (c) *lysA,* (d) *metF*, (e) VC0934 (coding for a glycosil transferase) and (f) *thyA.*
(a) To construct atoxigenic strains by inactivation of the cholera enterotoxin genes or deletion of the CTXΦ prophage, the suicide plasmid used was pJAF (Benitez y cols, 1996, Archives of Medical Research, Vol 27, No 3, pp. 275-283). This plasmid was obtained from plasmid pBB6 (Baudry y cols, 1991, Infection and Immunity 60:428), which contains a 5,1 kb insert from *V. cholerae* 569B that encodes *ace*, *zot*, *ctxA* y *ctxB*. Due to the absence of RS1 sequences 3' to the *ctxAB* operon in Classical vibrios, the *Eco*R I site downstream to the *ctxAB* copy in this plasmid lies in the flanking DNA of undefined function. The plasmid pBB6 was modified by deletion of the Scal internal fragment to create plasmid pBSCT5, which now contains a recombinant region deprived of the *zot* and *ctxA* functional genes. Then the *Pst*l of pBSCT5 was mutated into *Eco*RI by insertion of an *Eco*RI linker to obtain pBSCT64 and the resultant *Eco*RI fragment was subcloned into the *Eco*RI site of pGP704 to obtain pAJF.
(b) To construct strains affected in the expression of HA/P the suicide plasmid pGPH6 was used. This plasmid was constructed in different steps. First, plasmid pCH2 (Hase y Finkelstein, 1991, J. Bacteriology 173:3311-3317) that contains the *hap* gene in a 3,2 kb *Hin*dIII fragment from *V. cholerae* 3083 was linealized by the *Stu*I site, which is situated in the *hap* coding sequence. The 3.2 kb *Hind*III-fragment containing the *celA* gene was excised from plasmid pCT104 (Cornet y cols, 1983, Biotechnology 1:589 - 594) and subcloned into the Stul site of pCH2 to obtain pAHC3. The insert containing of pAHC3, containing the *hap* gene insertionally inactivated with the *c*e*lA* gene, was subcloned as a *Hin*dIII fragment to a pUC19 derivative that have the multiple cloning site flanked by *Bg*III sites to obtain plJHCI. The *Bgl* II fragment of this plasmid was subcloned into pGP704 to originate pGPH6, which contains a 6.4 kb fragment with the genetic *hap::celA* structure, where the *hap* gene is not functional.
(c) y (d) When constructing mutants in the *lysA* or *metF* genes, the suicide plasmids pCV*ly*s*A*Δ1 or pCVMΔClal were used. To construct these plasmids, the *lysA* y *metF* genes were PCR amplified from *V. cholerae* C7258, using a pair of oligonucleotides for each gene. The nucleotides sequences were purchased from Centro de Ingenieria Genética y Biotecnologia, Ciudad de La Habana, Cuba. The nucleotide sequesnces of the primers were: (*lysA*): (P 6488) 5'-GTA AAT CAC GCT ACT AAG-3' and (P 6487) 5'-AGA AAA ATG GAA ATGC-3' and (*metF*)*:* (P 5872) 5'-AGA GCA TGC GGC ATG GC-3' and (P 5873) 5'-ATA CTG CAG CTC GTC GAA ATG GCG-3'. The amplicons were cloned into the plasmids pGEM^{®}T (Promega) and plJ2925 (Janssen y cols, 1993, Gene 124:133-134), leading to the obtainment of the recombinant plasmids pGlysA3 y pMF29, which contain active copies of the *lysA* y *metF* genes, respectively. The identity of each gene was checked by nucleotide sequencing.
   The *metF* and *lysA* genes cloned were mutated *in vitro* by deletion of the respective *Cla*I (246 base pairs) and *Pst*I/*Acc*I (106 base pairs) inner fragments, respectively. In the last of the cases the strategy was designed to keep the open reading frame leading to an inactive gene product to avoid exerting polar effects during and after construction of a *lysA* mutant of *V. cholerae.* Each inactivated gen was cloned as a *Bgl* II fragment in the suicide vector pCVD442 for the subsequent introduction into the cholera vaccine candidates of interest. The suicide plasmid containing the *lysA* alelle was termed pCV*lysA*Δ1 and the one containing the *metF* alelle was denominated pCVMΔClal.
(e) When constructing mutants of the VC0934 gene we constructed and used the suicide plasmid pCVDΔ34. In doing that, the VC0934 gene was PCR amplified using as template total DNA from strain N16961 and the primers: 5'-GCA TGC GTC TAG TGA TGA AGG-3' y 5'-TCT AGA CTG TCT TAA TAC GC-3'. The amplicon was cloned into the plasmid pGEM5Zf T-vector to obtain plasmid pGEM34; a 270 base pair deletion was performed inside the VC0934 coding sequence using the restriction enzymes *Nar*I*lBgl*II. After flushing the ends with klenow and subsequent recircularization the plasmid obtained was named pG34. The resultant inactive gene was subcloned into the suicide vector pCVD442 digested with *Sal*I and *Sph*I to obtain the plasmid pCVΔ34. This plasmid was used to make the allelic replacement of the wild type gene.
(f) When constructing mutants defective in *thyA* expression the suicide plasmid pEST was constructed and used. The steps to construct this plasmid comprised the cloning of the *thyA* gene from *V. cholerae* C7258 into pBR322 as an *Eco*RI-*Hind*III cromosomal DNA fragment, to obtain pVT1 (Valle y cols, 2000, Infection and Immunity 68, No 11, pp6411-6418). A 300 base pairs internal fragment from the *thyA* gene, comprised between the B*gl*II and Mlul, sites was deleted from this plasmid to obtain pVMT1. This deletion removed the DNA fragment that codes for amino acids 7 to 105 of the encoded protein Thimidilate syntase. The mutated *thyA* gene was excised as an *Eco*RI-*Hind*III fragment, the extremes were blunted and then cloned into the *Sma*I site of pUC19 in the same orientation as the β-galactosidase gene to obtain pVT9. The resultant gene was subcloned as a *Sac*I fragment from pVT9 to pCVD442 and the obtained plasmid was named pEST. This final construct was used to make the allelic replacement of the wild type gene in the strains of interest.

The described suicide vectors are a modular system that can be used to introduce secuencial mutation into *V. cholerae* vaccine candidates.

The allelic replacement with the genes encoded by these vectors is done following the sequence of steps denoted below:
In the first step the suicide vector, containing the allele of choice among those described, is transferred by conjugation from the *E. coli* donor SM10λpir to the *V. cholerae* recipient, this last being the subject of the planned modification. This event is done to produce a cointegrate resistant to ampicillin. The clones resultant from the conjugational event are thus selected in LB plates supplemented with ampicillin (100 µg/ml).
The procedure for this first stage is as follows. The donor strain, SM10λpir transformed with the sucide vector of interest, is grown in an LB plate (NaCl, 10 g*l*l; bacteriological triptone, 10 g*l*l, and yeast extract, 5 g*l*l), supplemented with ampicillin (100 µg*l*ml), and the receptor strain, the *V. cholerae* strain to be modified, is grown in an LB plate. The conditions for growth are 37°C overnight. A single colony of the donor and one from the receptor is streaked into a new LB plate. The donor strain is streaked firstly in one direction and the receptor (*V. cholerae*) secondly in the opposed orientation. This perpendicular and superimposed streaking warrant that both strain grow in close contact. In the next step the plates are incubated at 37°C for 12 hours, harvested in 5 ml of NaCl (0.9 %) and 200 µl of dilutions 10², 10³, 10⁴ and 10⁵ are disseminated in LB-ampicillin-polimixinB plates, to select the *V. cholerae* clones that were transformed with the suicide plasmid and counterselect the donor *E. coli* SM10λpir. Ten such clones resulting from each process are preserved frozen at - 80°C in LB-glicerol at 20 % to be analyzed in the second step.
In the second step, a Southern blot hybridization is performed with a probe specific for the gene subjected to the mutational process; this is done to detect the structure of the correct cointegrate among the clones conserved in the previous step. The clones in which the suicide plasmid integrated to the correct target by homologous recombination are identified by the presence of a particular cromosomal structure. This structure contains one copy of the wild type gene and one of the mutated allele separated only by plasmid vector sequences. This particular structure produces a specific hybridization pattern in Southern blot with the specific probe that allows its identification. The appropriate clones are conserved frozen at -80°C in LB glicerol.
This second step comprises the following substeps: Firstly, the total DNA of each clone obtained in the first step is isolated according to a traditional procedure (Ausubel y cols, Short protocols in Molecular Biology, third edition, 1992, unit 2.4, page 2-11, basic protocol). Total DNA from the progenitor strain is isolated as control. Then, the total DNA of the ten clones the progenitor strain is digested with the appropriate restriction enzymes, to be mentioned subsequently in the document. One µg of DNA are digested from each clone and the mother strain and later electrophoresed in parallel lanes of an agarose gel. The DNA content of the gel is blotted into membranes in alkaline conditions (Ausubel y cols, Short protocols in Molecular Biology, third edition, 1992, unit 2.9 A, page 2-30, alternate protocol 1).

The blots are fixed by incubation at 80°C for 15 minutes. The free sites in the membrane are then blocked by prehybridization and subsequently probed with the specific probe for each mutation.

What follows are the details of the restriction enzyme, the probe (digoxigenin-labelled using the method random primed method) and the size of the hibridization fragment that identify the desired structure for the cointegrate of each clone, according to the target gene:
a) For suppression mutants of the CTXΦ phage genes, the total DNA of clones is digested with the restriction enzyme *Hind* III, and once in the membrane is hybridized with a probe obtained starting from the *Pst* I - *EcoR* I fragment of the pBB6 plasmid. The clones of interest are the ones that have the genetic structure that origin two bands in the Southern blot, one of 10 000 base pairs and another of 7 000 base pairs. As control the parental strain origins a single band of 17 000 base pairs in the same experiment of Southern blot.
b) For suppression mutants of the *hap* gene, the total DNA of clones is digested with the restriction enzyme *Xho* I, and once in the membrane is hybridized with a probe obtained starting from the *Hind* III fragment of 3 200 base pairs presents in the pCH2 plasmid. The clones of interest are those that have the genetic structure that origins a single band in the Southern blot, of 16 000 base pairs. As control the parental strain generates a single band of 6 000 base pairs in the same experiment of Southern blot.
c) For suppression mutants of *lysA* gene, the total DNA of clones is digested with the restriction enzyme *Xho* I, and once in the membrane is hybridized with a probe obtained from the *Sph* I/Sma I fragment of the pCVlysAD1 plasmid, contained the mutated gene *lysA*. The clones of interest are those that have the genetic structure that origins a single band in the Southern blot, of 12 500 base pairs. As control the parental strain generates a single band of 5 200 base pairs in the same experiment of Southern blot.
d) For suppression mutants of *metF* gene, the total DNA of clones is digested with the restriction enzyme *Nco* I, and once in the membrane is hybridized with a probe obtained from the *Bgl* II fragment of pCVDClal contained the mutated *metF* gene. The clones of interest are those that have the genetic structure that origins a single band in the Southern blot, of 12 000 base pairs. As control the parental strain generates a single band of 5 000 base pairs in the same experiment of Southern blot.
e) For suppression mutants of gene VC0934, the total DNA of clones is digested with the restriction enzyme *Ava* I, and once in the membrane is hybridized with a probe obtained from the *Sal* I/Sph I fragment of pcVDD34 contained the mutated VC0934 gene. The clones of interest are those that have the genetic structure that origins two bands in the Southern blot, one of 1 600 or 1900 and another of 8 200 or 7 900 base pairs. As control the parental strain generates a single band of 3 500 base pairs in the same experiment of Southern.
f) For suppression mutants in *thyA* gene, the total DNA of clones is digested with the restriction enzyme *Bstx* I, and once in the membrane is hybridized with a probe obtained from the *Sac* I fragment of pEST1, contained the mutated *thyA* gene. The clones of interest are those that have the genetic structure that origins a single band in the Southern blot, of 9 600 base pairs. As control the parental strain generates a single band of 2 400 base pairs in the same experiment of Southern blot.

In the third step of the procedure, 3 clones of interest, carrying a cointegrate with one of the previous structures, are cultured in absence of the antibiotic selective pressure to allow the loss of the suicidal vector by means of homologue recombination and the amplification of resultants clones. In said clones the loss of the suicidal vector goes with the loss of one of the two copies of the gene, the mutated or the wild one, of the genetic endowment of the bacteria.

In a fourth step of the procedure, dilutions of the previous cultures are extended in plates to obtain isolated colonies, which are then replicated toward plates supplemented with ampicillin to evaluate which clones are sensitive to ampicillin. Said clones, sensitive to ampcillin, are conserved for freezing, as described previously.

In a fifth step, by means of a study of Southern blot with specific probes for each one of the genes of interest (describe in a, b, c, d, and, f) it is verified which clones retained in the chromosome the mutated copy of the allele of interest. These clones of interest are expanded to create a work bank and to carry out their later characterization, as well as the introduction of the modifications object of protection in the present invention application.

In the following paragraphs we detail the restriction enzyme, the probe and the sizes of the hybridization fragments that identify the desired structure in each of the mutants, according to each of the genes being the subject of modification:
a) To analyze the mutants in the CTXΦ prophage, the total DNA is digested with the restriction endonuclease *Hin*d III. Once in the membrane it is hybridized with a probe derived from the *Pst* I - *Eco*R I fragment of plasmid pBB6. Are clones of interest such that do not produce hybridization bands in the Southern blot.
b) For the mutants with the inactivated allele of *hap*, total DNA from the clones is digested with the restriction enzyme *Xho* I and once in the membrane it is hybridized with a probe derived from the 3 200 base pair *Hind* III fragment from plasmid pCH₂ that codes for the *hap* gen. The clones of interest are those that produce a single band in the Southern blot, of about 9 000 nucleotide pairs.
c) For the mutants in the lysA gene total ADN is digested with the restriction enzyme *Xho* I, and once in the membrane it is hybridized with a probe derived from the *Sph* I*lSma* I fragment isolated from the plasmid pCVΔ*lysA*, that contain the *lysA* mutated gene. The clones of interest are those having the genetic structure that produce a single band in Southern blot of about de 5 000 pairs of nucleotides.
d) For the mutants with deletions in the *mefF* gene, the total ADN of the clones is digested with the restriction enzyme *Nco* I, and once in the membrane it is hybridized with a probe derived from the *Bg*/ II fragment contained in plasmid pCV*M*Δ*Cla*I, that contains the *metF* mutant gene. The clones of interest are those that have the genetic structure that leads to a single band of 4 700 base pairs in the Southern blot.
e) For the mutants in the VC0934 gene, total DNA of the clones is digested with the restriction enzyme *Ava* I, and the blots are hybridized with a probe obtained from the *Sal* I*lSph* I Fragment of pCVDΔ34, which contains the VC0934 mutant gene obtained *in vitro*. The clones of interest are those having the structure leading to a single band of 3 200 base pairs in the Southern blot.
f) For the mutants in the *thyA* gene, total DNA of the clones is digested with the restriction enzyme *Bstx* I, and the blots are hybridized with a probe obtained from the Sac I fragment of pEST1, which contain the *thyA* gene. The clones of interest are those that have the genetic structure leading to a single band in the Southern blot of about 2 100 base pairs.

### Example 7. Methods to preserve vaccine strains by means of lyophilization.

In following example microorganisms were cultured in LB broth at 37°C with an orbital shaking 150 and 250 rpm until reaching the logarithmic phase. Cells were harvested by centrifugation 5000 and 8000 rpm at 4°C during 10-20 minutes and then were mixed with the formulations that show good protection features of the microorganism, so that the cellular concentration was between 10⁸ and 10⁹ cells ml⁻¹. 2ml were dispensed for each 10R type flask. The lyophilization cycle comprised a deep freezing of the material, a primary drying keeping each product between -30°C and -39°C for space of 8 to 12 hours and a secondary drying at temperatures between 18°C and 25°C for not more than 12 hours. The viability loss was defined as the logarithmic difference of the CFU/mL before and after the lyophilization or before and after the storage of the lyophilized material, which is always dissolved in a 1.33% sodium bicarbonate solution.

### Formulation L+E+S

The BLR01, JCG03 and ESP05 strains were processed by the previously described lyophilization process in a formulation of the type L (5.0%), E (2.0%) and S (2.0%). The freezing was performed at -60°C. During the primary drying, the temperature of the product was kept at -32°C for 10 hours and in the secondary drying the temperature was kept at 22°C for 12 hours. The dissolution of the lyophilized material in a 1.33% sodium bicarbonate solution was instant. The viability loss calculated immediately after the dissolution, with regard to the concentration of live cells before the lyophilization resulted to be 0.30, 0.43, and 0.60 logarithmic orders for BLR01, JCG03 and ESP05, respectively.

### Comparison of the L+P+S and L+E+S formulations with that of skim milk+peptone +sorbitol.

The strain JCG03 was lyophilized using two formulations: the type L (6.0%), P (2.0%) and S (2.0%), and the other type L (5.5%), E (1.8%) and S (1.6%). This strain was also lyophilized in a formulation of 6.0% skim milk, 2.0% peptone and 2.0% sorbitol as a comparison formulation. The freezing was done at -60°C. During the primary drying, the temperature of the product was kept at -33°C for 12 hours and in the secondary drying the temperature was kept at 20°C for 14 hours. The dissolution of the lyophilized material in a 1.33% sodium bicarbonate solution was instant when the lyophilization process took place in the formulations of the type L+P+S or L+E+S and slightly slower when was lyophilized in the comparison formulation. The viability loss calculated immediately after the dissolution, with regard to the concentration of live cells before the lyophilization resulted to be 0.48, 0.52 and 0.55 logarithmic orders for the L+P+S, L+E+S and the comparison formulations, respectively, significantly similar.

### Humidity and oxygen effects

The strain JCG03 lyophilized in the three formulations mentioned in the previous paragraph, was exposed immediately after being lyophilized to the simultaneous action of humidity and oxygen. This was achieved, confining the samples during 3 days at 25°C in an atmosphere in sterile glass desiccators, under an 11 % relative humidity (created by a saturated solution of lithium chloride). The viability loss in the L+P+S, L+E+S and comparison formulations resulted to be 1.61, 1.10 and 3.43 logarithmic orders, respectively, what shows that the formulations object of this invention guarantee a bigger protection to humidity and oxygen than the comparison formulation.

### Effect of the storage temperature.

The strains TLP01, JCG01 and ESP05 were lyophilized in a formulation of the type L (5.5%), E(2.0%) and S(2.0%). The freezing was done at -58°C. During the primary drying, the temperature of the product was kept at -30°C for 12 hours and in the secondary drying the temperature was kept at 20°C for 14 hours. The dissolution in a 1.33% sodium bicarbonate solution was instant. The viability loss calculated immediately after the dissolution, with regard to the concentration of live cells before the lyophilization resulted to be 0.43, 0.55 and 0.44 logarithmic orders in TLP01, JCG01 and ESP05, respectively. The lyophilized material was stored 1 year either at 8°C or - 20°C. The Table 3 shows the viability loss results obtained.

**Table 3. Viability loss (1 year of storage).**

| Strain | 8°C | - 20°C |
|---|---|---|
| TLP01 | 1.07 | 0.64 |
| JCG01 | 1.02 | 0.59 |
| ESP05 | 0.91 | 0.55 |

### Example 8. Strains of the present invention and their characteristics

The strains of the present invention have been deposited in the Belgium Coordinated Collection of Microorganisms (BCCM). Laboratorium voor Microbiologie - Bacterienverzameling (LMG):
*Vibrio cholerae* JCG01 (LMG P-22149)
*Vibrio* cholerae JCG02 (LMG P-22150)
*Vibrio* cholerae JCG03 (LMG P-22151)
*Vibrio* cholerae KMD01 (LMG P-22153)
*Vibrio* cholerae KMD02 (LMG P-22154)
*Vibrio* cholerae KMD03 (LMG P-22155)
*Vibrio* cholerae JCG04 (LMG P-22152)
*Vibrio* cholerae ESP01 (LMG P-22156)
*Vibrio* cholerae ESP02 (LMG P-22157)
*Vibrio* cholerae ESP03 (LMG P-22158)
*Vibrio* cholerae RAF01 (LMG P-22159)
*Vibrio* cholerae TLP01 (LMG P-22160)
*Vibrio* cholerae TLP02 (LMG P-22161) and
*Vibrio* cholerae TLP03 (LMG P-22162)

They are described in Table 4.

**Table 4. Vaccine strains of the present invention.**

| Strain | Wild type parental strain | Biotype/Serotype | Relevant Genotype. |
|---|---|---|---|
| BLR01 | CA385 | Classical*l*Ogawa | ΔCTXΦ, *hap::celA*, Δ*mshA* |
| BLR02 | CA385 | Classical/Ogawa | ΔCTXΦ, *hap::celA, lysA*, Δ*mshA* |
| BLR03 | CA385 | Classical/Ogawa | ΔCTXΦ, *hap::celA*, *metF*, Δ*mshA* |
| EMG01 | CA401 | Classical*l*lnaba | ΔCTXΦ, *hap::celA*, Δ*mshA* |
| EMG02 | CA401 | Classical*l*lnaba | ΔCTXΦ, *hap::celA*, *lysa*, Δ*mshA* |
| EMG03 | CA401 | Classical*l*lnaba | ΔCTXΦ, *hap::celA*, *metF*, Δ*mshA* |
| JCG01 | C7258 | El Tor/Ogawa | ΔCTXΦ, *hap::celA*, MSHA⁻ |
| JCG02 | C7258 | El Tor/Ogawa | ΔCTXΦ, *hap::celA*, MSHA⁻ |
| JCG03 | C7258 | El Tor/Ogawa | ΔCTXΦ, *hap::ce*/*A*, Δ*mshA* |
| EVD01 | C7258 | El Tor/Ogawa | ΔCTXΦ, *hap::celA*, *thyA*, Δ*mshA* |
| KMD01 | C7258 | El Tor/Ogawa | ΔCTXΦ, *hap::celA*, *metF*, Δ*mshA* |
| KMD02 | C7258 | El Tor/Ogawa | ΔCTXΦ, *hap*::*celA*, *lysA*, Δ*mshA* |
| ESP06 | C7258 | El Tor/Ogawa | DCTXF, *hap*:: *celA*, DVCO934, *DmshA* |
| JCG04 | C7258 | El Tor/Ogawa | ΔCTXΦ, *hap*::*celA*, Δ*mshA* |
| ESP01 | C7258 | El Tor/Ogawa | ΔCTXΦ, *hap*::*celA*, *mefF*, Δ*mshA* |
| ESP02 | C7258 | El Tor/Ogawa | ΔCTXΦ, *hap*::*celA*, *lysA*, Δ*mshA* |
| ESP04 | C7258 | El Tor/Ogawa | DCTXF, *hap*:: *celA*, DVCO934, *DmshA* |
| RAF01 | C6706 | El Tor*l*lnaba | ΔCTXΦ, *hap*::*celA*, Δ*mshA* |
| EVD02 | C6706 | El Tor*l*lnaba | ΔCTXΦ, *hap*::*celA*, *thyA*, Δ*mshA* |
| ESP03 | C6706 | El Tor*l*lnaba | ΔCTXΦ, *hap*::*celA*, *metF*, Δ*mshA* |
| KMD03 | C6706 | El Tor*l*lnaba | ΔCTXΦ, *hap*::*celA*, *lysA*, Δ*mshA* |
| ESP05 | C6706 | El Tor/lnaba | DCTXF, *hap*:: *celA*, DVCO934, *DmshA* |
| TLP01 | CRC266 | 0139 | ΔCTXΦ, *hap*::*celA*, Δ*mshA* |
| TLP02 | CRC266 | 0139 | ΔCTXΦ, *hap*::*celA*, *lysA*, Δ*mshA* |
| TLP03 | CRC266 | 0139 | ΔCTXΦ, *hap*::*celA*, *metF*, Δ*mshA* |

### Brief description of the drawings.

Figure 1. Microphotography of VGJΦ phage. Magnification x 32 000. VGJΦ phage was purified from the supernatants of infected *Vibrio cholerae* 569B.
Figure 2. Diagram of the genome of hybrid phage HybPΦ-kn, which has a high potentiality for cholera toxin transmission.
Figure 3. Scheme of the genetic manipulation used to suppress *mshA* gene of *V*. *cholerae* vaccine candidates and the suicide vector used during the proceeding.
Figure 4. Suckling Mice survival inoculated with an attenuated strain and its derivative infected with HybPΦ-Kn that revert it to virulence.

### Advantages

The present invention provide us with a methodology to protect live cholera vaccine candidates from the reacquisition of cholera toxin genes and others toxins from the CTXΦ bacteriophage mediated by VGJΦ phage, and therefore from the conversion to virulence by this mechanism.

Equally provide us with the necessary information to assure that live cholera vaccine candidates will not spread CTXΦ, in the case that these vaccine candidates reacquire CTXΦ, by a specialized transduction with the VGJΦ phage.

The present invention provide us the application of MSHA mutants as live cholera vaccine candidates, which exhibit an increase in their environmental safety due to resistance to the infection with CTXΦ mediated by VGJΦ.

This invention provides us with a new characteristic to keep in mind during the design and construction of live cholera vaccine candidates to improve their environmental safety, that is to say that such vaccines are not able to spread the CTXΦ genes, mediated by VGJΦ, in the case of reacquisition.

The above characteristic could be applied to the already made live cholera vaccine candidates, which have demonstrated an acceptable level of reactogenicity in volunteers studies, to reduce their potential environmental impact.

This invention also provides formulations to preserve by lyophilization all of the above-mentioned live cholera vaccine candidates and also improve their abilities to tolerate the remainder of oxygen and humidity in the container.

These formulations also guarantee the instant reconstitution of the lyophilized live cholera vaccine candidate powder in sodium bicarbonate buffer, making easier the manipulation, protecting the vaccines during this process and improving the organoleptic characteristics, specifically related with the visual aspect of lyophilized tablets and the reconstituted products.

One of the formulations provided here for conservation and lyophilization of live cholera vaccine candidates lacks the bovine components usually added to many formulations to lyophilize human vaccines.

## Claims

1. Live attenuated strains of *Vibrio cholerae* **characterized in**
- lacking of sequences of the DNA sequence of a filamentous bacteriophage isolated form Vibrio cholerae O139, termed phage VGJΦ (SEC ID: No 1) in their genomes to impair the formation of the hybrid recombinant phage VGJΦ::CTXΦ and prevent further spreading of the cholera toxin genes of CTXΦ via this new phage, and
- having at least one mutation comprising the inactivation of one gene essential for the biogenesis of MSHA, the receptor to phage VGJΦ, or a deletion introduced in the mshA gene coding for the structural subunit of MSHA fimbria, in order to prevent the reversion to virulence of said strains by infection with the hybrid recombinant phage VGJΦ::CTXΦ;

2. Live attenuated strains of Vibrio cholerae, according to claim 1, in which said mutation is a spontaneous mutation.

3. Live attenuated strains of Vibrio cholerae, according to claim 1 or 2, coming from one or more of the following strains of Vibrio cholerae (serogroup O1 or 139, biotipe El Tor and serotipe Ogawa or Inaba), deposited with the Belgium Coordinate Collection of Microorganisms (BCCM), Laboratorium Voor Microbiologie-Bacterienverzameling (LMG):
Vibrio cholerae JCG01 (LMG P-22149), serogroup 01, biotype El
Tor, serotype Ogawa,
Vibrio cholerae JCG02 (LMG P-22150), serogroup 01, biotype El
Tor, serotype Ogawa,
• Vibrio cholerae JCG03 (LMG P-22151), serogroup O1, biotype El Tor, serotype Ogawa,
• Vibrio cholerae KMD01 (LMG P-22153), serogroup O1, biotype El Tor, serotype Ogawa,
• Vibrio cholerae KMD02 (LMG P-22154), serogroup O1, biotype El Tor, serotype Ogawa,
• Vibrio cholerae KMD03 (LMG P-22155), serogroup O1, biotype El Tor, serotype Inaba,
• Vibrio cholerae JCG04 (LMG P-22152), serogroup O1, biotype El Tor, serotype Ogawa,
• Vibrio cholerae ESP01 (LMG P-22156), serogroup O1, biotype El Tor, serotype Ogawa,
• Vibrio cholerae ESP02 (LMG P-22157), serogroup O1, biotype El Tor, serotype Ogawa,
• Vibrio cholerae ESP03 (LMG P-22158), serogroup O1, biotype El Tor, serotype Inaba,
• Vibrio cholerae RAF01 (LMG P-22159), serogroup O1, biotype El Tor, serotype Inaba,
• Vibrio cholerae TLP01 (LMG P-22160), serogroup O139,
• Vibrio cholerae TLP02 (LMG P-22161), serogroup O139,
• Vibrio cholerae TLP03 (LMG P-22162), serogroup 0139.

4. Freeze dried formulations comprising live attenuated strains of Vibrio cholerae, according to any of claims 1-3, and a combination of lactose, sorbitol and peptone or lactose, sorbitol and yeast extract, at a total concentration not higher than 10%.

5. Freeze dried formulations, according to claim 4, comprising live attenuated strains of Vibrio cholerae and a combination of 6.0% lactose, 2.0% peptone, and 2.0% sorbitol or 5.0% lactose, 2.0% yeast extract and 2.0% sorbitol.

6. Use of the live attenuated Vibrio cholerae strains, according to any of claims 1-3, in the manufacture of live attenuated cholera oral vaccines of improved environmental safety.

7. Use of the freeze dried formulations, according to claims 4 or 5, in the manufacture of live attenuated cholera oral vaccines of improved environmental safety.

## Patentansprüche

1. Lebende abgeschwächte *Vibrio cholerae-Stämme*, **dadurch gekennzeichnet, dass**
- in ihren Genomen die Sequenzen der DNA-Sequenz eines filamentösen Bakteriophagen, welcher aus Vibrio cholerae 0139 isoliert und Phage VGJΦ (SEQ ID NO: 1) genannt wurde, fehlen, um die Bildung des hybriden, rekombinanten Phagen VGJΦ::CTXΦ zu vermindern und weiteres Ausbreiten der Choleratoxin-Gene von CTXΦ mittels dieses neuen Phagen zu verhindern, und
- sie mindestens eine Mutation haben, welche die Inaktivierung eines Gens umfasst, welches grundlegend für die Biogenese von MSHA ist, dem Rezeptor für den Phagen VGJΦ, oder eine Deletion haben, welche in das mshA Gen eingeführt wurde, welches für die strukturelle Untereinheit der MSHA-Fimbrie kodiert, um die Rückkehr zur Virulenz der Stämme durch Infektion mit dem hybriden, rekombinanten Phagen VGJΦ::CTXΦ zu verhindern.

2. Lebende abgeschwächte Vibrio cholerae-Stämme gemäß Anspruch 1, wobei die Mutation eine spontane Mutation ist.

3. Lebende abgeschwächte Vibrio cholerae-Stämme gemäß Anspruch 1 oder 2, wobei sie aus einem oder mehreren der folgenden Vibrio cholerae-Stämme (Serogruppe O1 oder 139, Biotyp El Tor und Serotyp Ogawa) oder Inaba) stammen, hinterlegt bei der Belgium Coordinate Collection of Microorganisms (BCCM), Laboratorium Voor Microbiologie-Bacterienverzameling (LMG):
• Vibrio cholerae JCG01 (LMG P-22149), Serogruppe O1 Biotyp El Tor, Serotyp Ogawa,
• Vibrio cholerae JCG02 (LMG P-22150), Serogruppe O1, Biotyp El Tor, Serotyp Ogawa,
• Vibrio cholerae JCG03 (LMG P-22151), Serogruppe O1, Biotyp El Tor, Serotyp Ogawa,
• Vibrio cholerae KMD01 (LMG P-22153), Serogruppe O1, Biotyp El Tor, Serotyp Ogawa,
• Vibrio cholerae KMD02 (LMG P-22154), Serogruppe O1 Biotyp El Tor, Serotyp Ogawa,
• Vibrio cholerae KMD03 (LMG P-22155), Serogruppe O1, Biotyp El Tor, Serotyp Inaba,
• Vibrio cholerae JCG04 (LMG P-22152), Serogruppe O1, Biotyp El Tor, Serotyp Ogawa,
• Vibrio cholerae ESP01 (LMG P-22156), Serogruppe O1, Biotyp El Tor, Serotyp Ogawa,
• Vibrio cholerae ESP02 (LMG P-22157), Serogruppe O1, Biotyp El Tor, Serotyp Ogawa,
• Vibrio cholerae ESP03 (LMG P-22158), Serogruppe O1, Biotyp El Tor, Serotyp Inaba,
• Vibrio cholerae RAF01 (LMG P-22159), Serogruppe O1 Biotyp El Tor, Serotyp Inaba,
• Vibrio cholerae TLP01 (LMG P-22160), Serogruppe 0139,
• Vibrio cholerae TLP02 (LMG P-22161), Serogruppe 0139,
• Vibrio cholerae TLP03 (LMG P-22162), Serogruppe 0139.

4. Gefriergetrocknete Formulierungen, welche lebende abgeschwächte Vibrio cholerae-Stämme gemäß einem der Ansprüche 1-3 und eine Kombination von Laktose, Sorbitol und Pepton oder Laktose, Sorbitol und Hefeextrakt mit einer Gesamt-Konzentration von nicht höher als 10% umfassen.

5. Gefriergetrocknete Formulierungen gemäß Anspruch 4, welche lebende abgeschwächte Vibrio cholerae-Stämme und eine Kombination von 6,0%iger Laktose, 2,0%igem Pepton und 2,0%igem Sorbitol oder 5,0%iger Laktose, 2,0%igem Hefeextrakt und 2,0%igem Sorbitol umfassen.

6. Verwendung der lebenden abgeschwächten Vibrio cholerae-Stämme gemäß einem der Ansprüche 1-3 bei der Herstellung von oralen, lebenden abgeschwächten Cholera-Vakzinen von verbesserter Umweltsicherheit.

7. Verwendung der gefriergetrockneten Formulierungen gemäß Anspruch 4 oder 5 in der Herstellung von oralen, lebenden abgeschwächten Cholera-Vakzinen von verbesserter Umweltsicherheit.

## Revendications

1. Souches vivantes atténuées de Vibrio cholerae,
**caractérisées en ce qu'**
- elles ne contiennent pas les séquences de la séquence d'ADN d'un bactériophage filamenteux isolé à partir de Vibrio cholerae 0139, appelé phage VGJΦ (SEC ID : No 1) dans leur génome afin d'entraver la formation du phage recombinant hybride VGJΦ::CTXΦ et de prévenir la prolifération des gènes de la toxine cholérique du CTXΦ par le biais de ce nouveau phage, et
- elles ont au moins une mutation comprenant l'inactivation d'un gène essentiel à la biogenèse du MSHA, le récepteur du phage VGJΦ, ou une délétion introduite dans le gène mshA codant pour la sous-unité structurale de la fimbria MSHA, afin de prévenir la réversion de la virulence desdites souches par infection avec le phage recombinant hybride VGJΦ::CTXΦ.

2. Souches vivantes atténuées de Vibrio cholerae, conformément à la revendication 1, dans lesquelles la mutation est une mutation spontanée.

3. Souches vivantes atténuées de Vibrio cholerae, conformément à la revendication 1 ou 2, provenant d'une ou de plusieurs des souches suivantes de Vibrio cholerae (sérogroupe O1 ou 139, biotype El Tor et sérotype Ogawa ou Inaba), déposées auprès de la Belgium Coordinate Collection of Microorganisms (BCCM), Laboratorium Voor Microbiologie-Bacterienverzameling (LMG) :
• Vibrio cholerae JCG01 (LMG P-22149), sérogroupe O1, biotype El Tor, sérotype Ogawa,
• Vibrio cholerae JCG02 (LMG P-22150), sérogroupe O1, biotype El Tor, sérotype Ogawa,
• Vibrio cholerae JCG03 (LMG P-22151), sérogroupe O1, biotype El Tor, sérotype Ogawa,
• Vibrio cholerae KMD01 (LMG P-22153), sérogroupe O1, biotype El Tor, sérotype Ogawa,
• Vibrio cholerae KMD02 (LMG P-22154), sérogroupe O1, biotype El Tor, sérotype Ogawa,
• Vibrio cholerae KMD03 (LMG P-22155), sérogroupe O1, biotype El Tor, sérotype Inaba,
• Vibrio cholerae JCG04 (LMG P-22152), sérogroupe O1, biotype El Tor, sérotype Ogawa,
• Vibrio cholerae ESP01 (LMG P-22156), sérogroupe O1 biotype El Tor, sérotype Ogawa,
• Vibrio cholerae ESP02 (LMG P-22157), sérogroupe O1, biotype El Tor, sérotype Ogawa,
• Vibrio cholerae ESP03 (LMG P-22158), sérogroupe O1, biotype El Tor, sérotype Inaba,
• Vibrio cholerae RAF01 (LMG P-22159), sérogroupe O1, biotype El Tor, sérotype Inaba,
• Vibrio cholerae TLPO1 (LMG P-22160), sérogroupe O139,
• Vibrio cholerae TLP02 (LMG P-22161), sérogroupe O139,
• Vibrio cholerae TLP03 (LMG P-22162), sérogroupe O139.

4. Formulations lyophilisées comprenant des souches vivantes atténuées de Vibrio cholerae, conformément à l'une quelconque des revendications 1 à 3, et une combinaison de lactose, de sorbitol et de peptone ou de lactose, de sorbitol et d'extrait de levure, dans une concentration totale ne dépassant pas 10 %.

5. Formulations lyophilisées, conformément à la revendication 4, comprenant des souches vivantes atténuées de Vibrio cholerae et une combinaison de lactose à 6,0 %, de peptone à 2,0 %, et de sorbitol à 2,0 % ou de lactose à 5,0 %, d'extrait de levure à 2,0 % et de sorbitol à 2,0 %.

6. Utilisation des souches vivantes atténuées de Vibrio cholerae, conformément à l'une quelconque des revendications 1 à 3, dans la fabrication de vaccins oraux vivants atténués contre le choléra montrant une innocuité environnementale améliorée.

7. Utilisation des formulations lyophilisées, conformément aux revendications 4 ou 5, dans la fabrication de vaccins oraux vivants atténués contre le choléra montrant une innocuité environnementale améliorée.
